# EUROPEAN PATENT SPECIFICATION

(11) **EP 1 440 149 B1**
(45) Date of publication and mention of the grant of the patent: **02.08.2006**
(21) Application number: 02797952.5
(22) Date of filing: 05.09.2002
(51) Int. Cl.: C12N 7/04

(54) **USE OF AN INFECTIOUS AND ATTENUATED TYPE II BOVINE VIRAL DIARRHEA VIRUS CLONE TO PREVENT FOETAL INFECTION BY TYPE I BVDV**
VERWENDUNG EINES KLONES VON INFEKTIÖSEN UND ATTENUIERTEN Typ II RINDERDIARRHÖVIREN UM FÖTAL INFEKTION VON TYP I BVDV ZU VERMEIDEN
L'UTILISATION D'UN VIRUS INFECTIEUX ET ATTENUE DE LA DIARRHEE VIRALE BOVINE TYPE II POUR LA PRÉVENTION D'INFECTION FOETAL PAR BVDV DE TYPE I

(30) Priority: 06.09.2001 DE 10143813
(43) Date of publication of application: 28.07.2004
(60) Divisional application: 06011790.0
(73) Proprietor: BOEHRINGER INGELHEIM VETMEDICA GMBH, 55216 Ingelheim am Rhein (DE)
(72) Inventor: ELBERS, Knut, 55435 GAU-ALGESHEIM (DE); MEYER, Christiane, 48163 MÜNSTER (DE); VON FREYBURG, Martina, 72076 TÜBINGEN (DE); MEYERS, Gregor, 72141 WALDDORFHAESLACH (DE)
(86) International application number: PCT/EP2002/009925
(87) International publication number: WO 2003/023041

(56) References cited:
- WO-A-01/39801
- MEYER CHRISTIANE ET AL: "Recovery of virulent and RNase-negative attenuated type 2 bovine viral diarrhea viruses from infectious cDNA clones." JOURNAL OF VIROLOGY, vol. 76, no. 16, August 2002 (2002-08), pages 8494-8503, XP002251604 August, 2002 ISSN: 0022-538X
- DATABASE EMBL [Online] retrieved from EMBL Database accession no. AF145967 XP002251610
- RIDPATH JULIA F ET AL: "The genomic sequence of a virulent bovine viral diarrhea virus (BVDV) from the type 2 genotype: Detection of a large genomic insertion in a noncytopathic BVDV." VIROLOGY, vol. 212, no. 1, 1995, pages 39-46, XP002251605 ISSN: 0042-6822
- ODEÓN A C ET AL: "Experimental infection of calves with bovine viral diarrhea virus genotype II (NY-93)." JOURNAL OF VETERINARY DIAGNOSTIC INVESTIGATION: OFFICIAL PUBLICATION OF THE AMERICAN ASSOCIATION OF VETERINARY LABORATORY DIAGNOSTICIANS, INC. UNITED STATES MAY 1999, vol. 11, no. 3, May 1999 (1999-05), pages 221-228, XP009015305 ISSN: 1040-6387
- TOPLIFF CHRISTINA L ET AL: "Virulence markers in the 5' untranslated region of genotype 2 bovine viral diarrhea virus isolates." VIROLOGY, vol. 250, no. 1, 10 October 1998 (1998-10-10), pages 164-172, XP002251606 ISSN: 0042-6822
- KUEMMERER BEATE M ET AL: "Correlation between point mutations in NS2 and the viability and cytopathogenicity of Bovine viral diarrhea virus strain Oregon analyzed with an infectious cDNA clone." JOURNAL OF VIROLOGY, vol. 74, no. 1, January 2000 (2000-01), pages 390-400, XP002251607 ISSN: 0022-538X
- MEYERS GREGOR ET AL: "Recovery of cytopathogenic and noncytopathogenic bovine viral diarrhea viruses from cDNA constructs." JOURNAL OF VIROLOGY, vol. 70, no. 12, 1996, pages 8606-8613, XP002251609 ISSN: 0022-538X
- VASSILEV V ET AL: "Authentic and full-length genomic cDNA clones of BVDV that yield infectious transcripts" JOURNAL OF VIROLOGY, THE AMERICAN SOCIETY FOR MICROBIOLOGY, US, vol. 71, no. 1, 1997, pages 471-478, XP002144456 ISSN: 0022-538X

## Description

### Field of the invention

The invention belongs to the field of animal health and in particular Bovine Viral Diarrhea Virus (BVDV). Described are infectious BVDV clones and methods to produce said BVDV clones. Further described are methods of attenuating said clones, attenuated BVDV clones and vaccines comprising said attenuated clones, and its use for the preparation of a medicament for the prevention of fetal infection after challenge with an heterologolis challenge virus belonging to the BVDV type I antigenic group.

### Background of the Invention

Bovine Viral Diarrhea Virus (BVDV) is the causative agent of BVD and mucosal disease in cattle (Baker, 1987; Moennig and Piagemann. 1992; Thiel et al., 1996). Fetal infection during pregnancy can result in the resorption of the fetus, abortions as well as birth of immunotolerant calves which are persistently infected with BVDV. These calves lack or have very low neutralizing antibody titers and are continuously shedding high amounts of virus. Next to acutely infected cattle these calves are the major source for virus spreading and are therefore of prime importance in the epidemiology of this disease. The major economical impact of BVD results from high abortion rates, stillbirths, fetal resorption, mummification, congenital malformations, and birth of weak and undersized calves. For a detailed review of the pathogenesis it is hereby referred to the article of Moennig and Liess of 1995.
Two major antigenic groups of BVDV (type 1 and 2) have been described (Becher et al, 1999) which display limited cross neutralizing antibody reactions (Ridpath et al. 1994). Present vaccines for the prevention and treatment of BVDV infections still have drawbacks (Oirschot et al. 1999). Vaccines against the classical BVDV type 1 provide only partial protection from type 2 infection, and vaccinated dams may produce calves that are persistently infected with virulent OVDV type 2 (Bolin et al., 1991. Ridpath et al., 1994). This problem is probably due to the great antigenic diversity between type 1 and type 2 strains which is most pronounced in the glycoprotein E2, the major antigen (Tijssen et al., 1996): most monoclonal antibodies against type 1 strains fail to bind to type 2 viruses (Ridpath et al., 1994).

Killed vaccines (inactivated whole virus) or subunit vaccines (conventionally purified or heterologously expressed purified viral proteins) are most often inferior to live vaccines in their efficacy to produce a full protective immune response even in the presence of adjuvants.
Live BVDV vaccines, although attenuated, are most often associated with safety problems. As mentioned above, they cross the placenta of pregnant cows and lead to clinical manifestations in the fetus and/or the induction of persistently infected calves. Therefore, they cannot be applied to breeding herds that contain pregnant cows. Pregnant cows have to be kept separate from vaccinated cattle to protect fetuses and must not be vaccinated fihemselves. Furthermore, revertants of attenuated live BVDV pose a serious threat to cattle. For conventionally derived attenuated viruses wherein the attenuation is achieved by conventional multiple passaging, the molecular origin as well as the genetic stability of the attenuation remains unknown and reversion to the virulent wild-type is unpredictable.
Live vaccines with defined mutations as a basis for attenuation would overcome the disadvantages of the present generation of attenuated vaccines. A further advantage of said attenuating mutations lies in their defined molecular uniqueness which can be used as a distinctive label for the attenuated pestivirus to distinguish it from pestiviruses from the field.
In the art, BVDV of defined genetic identity which closely resemble wild-type viruses are hardly known, in particular not for type 2 BVDV. In the art, there was a long lasting need for methods to generate such BVDV. Therefore, the technical problem underlying this invention was to provide a BVDV, in particular a BVDV type 2, of defined genetic identity.

### Figure Legends

FIG. 1. Construction of the infectious cDNA clone, The upper part scetches a BVDV genome (kB) and the encoded polyprotein. The middle part shows the cDNA clones (white), the RT-PCR product (light grey) and the PCR products (dark grey) used for engineering the infectious cDNA clone, and the lower part depicts the ends of the genomic cDNA sequences (underlined) and the sequences added at the 5' and 3' ends for in vitro transcription.
Fig. 2: Growth curves of the recombinant virus XIKE-A and the wild type BVDV isolate VLS#399. MDSK cells were infected with the viruses at an m.o.l of 0.1 and harvested by freezing and thawing at the indicated time points. Titers were determined after infection of new MDBK cells by immunofluorescence staining 72h p.i.
Fig. 3: Growth curves of the recombinant virus XIKE-A and the E^{rns} mutants XIKE-B (H349Δ) and XIKE-C (H300L). MDBK cells were infected with the viruses at an m.o.i of 0.1 and harvested by freezing and thawing at the indicated time points. Titers were determined after infection of new MDBK cells by immunofluorescence staining 72h p.i.
Fig. 4: Determination of RNAse activity of the recombinant viruses XIKE-A (wild type sequence), XIKE-B (H349Δ) and XIKE-C (H300L) in comparison with the wild type strain new York '93/C from crude cell extracts of MDBK cells infected with the respective viruses. MDBK cells that were not infected served as a negative control (n.i.). The enzymatic degradation of poly(U) was determined by measuring the OD₂₆₀ as a marker of the release of small RNA fragments into the supernatant.
Fig. 5: Body temperatures of animals infected with New York '93/C (animal #275, #612 and #1610, broken lines) or XIKE-A (animal #615, #377 and #091, solid lines).
Fig. 6: White blood cell (WBC) counts of animals infected with New York '93/C (animals #275, #612 and #1810, broken lines) or XIKE-A (animals #615, #377 and #091. solid lines).
Fig. 7: Body temperatures of animals infected with XIKE-A (animal #387, #388 and #418, broken lines) or XIKE-B (animal #415, #417 and #419, solid lines).
Fig. 8: White blood cell I(WBC) counts of animals infected with XIKE-A (animals #387, #388 and #418, broken lines) or XIKE-B (animals #415. #417 and #419, solid lines).

### Disclosure of the invention

### Definitions of terms used in the description:

Before the embodiments of the present invention it must be noted that as used herein and in the appended claims, the singular forms "a", "an", and "the" include plural reference unless the context clearly dictates otherwise. Thus, for example, reference to "a BVDV virus" includes a plurality of such BVDV viruses, reference to the "cell" is a reference to one or more cells and equivalents thereof known to those skilled in the art, and so forth. Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications mentioned herein are incorporated herein by reference for the purpose of describing and disclosing the cell lines, vectors, and methodologies as reported in the publications which might be used in connection with the invention. Nothing herein is to be construed as an admission that the invention is not entitled to antedate such disclosure by virtue of prior invention.

The term "BVDV" as used herein refers to all viruses belonging to species BVDV 1 and BVDV 2 in the genus pestivirus within the family Flaviviridae (Becher et al. 1999).
The more classical BVDV type 1 strains and the more recently recognized BVDV type 2 strains display some limited but distinctive differences in nucleotide and amino acid sequences.

A "clone" is a DNA vector or host cell strain into which such vector has been Introduced. Preferably, the DNA vector is a plasmid.

An "infectious clone" is a DNA Vector with the capability to serve as a template for transcription into a RNA that Induces the generation of the the virus when introduced into susceptible cells. Preferably the RNA is produced by *in vitro* transcription and introduced into the cells by transfection technologies known to the skilled person.

"BVDV particles" or "viral particles" as used herein relates to BVD viruses generated from "infectious clones" via RNA, that will induce production of said BVDV particles when introduced into susceptible cells.
"Attenuated BVDV particles" or "attenuated viral particles" as used herein relates to BVDV particles attenuated by a method as disclosed herein (*see infra).*

"Infectivity" is the capability of a virus or viral particle to induce a certain number of plaques in a plaque test or a certain TCID₅₀ score in an endpoint test.

A full-length RNA is a RNA comprising at least 98 % of the sequence of a RNA occurring in a wild-type isolate. A full-length complementary DNA is a DNA comprising a sequence complementary to at least 98 % of a RNA occuring in a wild-type isolate.

As used herein, ''calf" relates to a bovine animal of six months of age or less.

Virulence: "Authentical virulence'' as used herein means that there is no statistically significant difference between the virulence of infectious BVDV particles as disclosed herein and wild-type BVDV isolates from which said DNA molecules containing a nucleotide sequence complementary to a BVDV RNA, preferably a type 2 RNA have been derived, for at least one predominant clinical parameter. Examples for such predominant clinical parameters are diarrhea, pyrexia and/or lethalifiy.

Aftenuaffon: "An attenuated BVDV particle" as used herein means that there is a statistically significant difference between the virulence of attenuated BVDV particles as disclosed herein, said attenuated BVDV particles being attenuated by a method as disclosed herein, and wild-type BVDV isolates from which said attenuated BVDV particles have been derived, for the predominant clinical parameters diarrhea, pyrexia and lethality In animals infected with the same dose, preferably 6x10⁸TCID₅₀. Thus, said attenuated BVDV particles do not cause diarrhea, pyrexia and lethality and thus may be used in a vaccine.

"RACE" as used herein means rapid amplification of cDNA ends and is known as such in the art (Frohman et al, Proc. Natl. Acad. Sci USA 1988, 85; 8998-9002),

"Susceptible cell" as used herein is a cell which can be infected with BVD virus or transfected with BVDV RNA, wherein said virus or RNA, when introduced into said susceptible cells, induces the generation of Infectious BVDV.

A "fragment" means any subunit of a DNA molecule or Infectious BVDV clone as disclosed herein, i.e. any subset, characterized in that it is encoded by a shorter nucleic acid molecule than disclosed which can still be transcribed into RNA.
A "functional variant" of the DNA molecule or infectious BVDV clone means a DNA molecule or infectious BVDV clone which possesses a biological activity (either functional or structural) that is substanfiially similar to the DNA molecule or infectious BVDV clone as disclosed herein. The term "functional variant" also includes a fragment", "a functional variant", "variant based on the degenerative nucleic acid code" or "chemical derivative". Such a functional variant" e.g. may carry one or several nucleic acid exchanges, deletions or insertions. Said exchanges, deletions or insertions may account for 10% of the entire sequence. Said functional variant at least partially retains its biological activity, e.g. function as an infectious clone or a vaccine strain, or even exhibits improved biological activity.
A "variant based on the degenerative nature of the genetic code" is a variant resulting from the fact that a certain amino acid may be encoded by several different nucleotide tripletts. Said variant at least partially retains its biological activity, or even exhibits improved biological activity,
A "fusion molecule" may be the DNA molecule or infectious BVDV clone as disclosed herein fused to e.g. a reporter such as a radiolabel, a chemical molecule such as a fluorescent label or any other molecule known in the art.
As used herein, a "chemical derivative" means a DNA molecule or infectious BVDV clone as disclosed herein chemically modified or containing additional chemical moieties not normally being part of the molecule, Such moieties may improve the molecule's solubility, absorption, biological half life etc.

A molecule is "substantially similar" to another molecule if both molecules have substantially similar nucleotide sequences or biological activity. Thus, provided that two molecules possess a similar activity, they are considered variants as that term is used herein if the nucleotide sequence is not identical, and two molecules which have a similar nucleotide sequence are considered variants as that term is used herein even if their biological activity is not identical.

The term ''vaccine'' as used herein refers to a pharmaceutical composition comprising at least one immunologically active component that induces an immunological response in an animal and possibly but not necessarily one or more additional components that enhance the immunological activity of said active component. A vaccine may additionally comprise further components typical to pharmaceutical compostions. The immunologically active component of a vaccine may comprise complete virus particles in either their original form or as attenuated particles in a so called modified live vaccine (MLV) or particles inactivated by appropriate methods in a so called killed vaccine (KV). In another form the immunologically active component of a vaccine may comprise appropriate elements of said organisms (subunit vaccines) whereby these elements are generated either by destroying the whole particle or the growth cultures containing such particles and optionally subsequent purification steps yielding the desired structure(s), or by synthetic processes including an appropriate manipulation by use of a suitable system based on, for example, bacteria, insects, mammalian or other species plus optionally subsequent isolation and purification procedures, or by induction of said synthetic processes in the animal needing a vaccine by direct incorporation of genetic material using suitable pharmaceutical compositions (polynucleotide vaccination). A vaccine may comprise one or simultaneously more than one of the elements described above.
The term "vaccine" as understood herein is a vaccine for veterinary use comprising antigenic substances and is administered for the purpose of inducing a specific and active immunity against a disease provoked by BVDV. The BVDV done as disclosed herein confers active immunity that may be transferred passively via maternal antibodies against the immunogens it contains and sometimes also against antigenically related organisms.
Additional components to enhance the immune response are constituents commonly referred to as adjuvants, like e.g. aluminiumhydroxide, mineral or other oils or ancillary molecules added to the vaccine or generated by the body after the respective induction by such additional components, like but not restricted to interferons, interleukins or growth factprs.
A "pharmaceutical composition" essentially consists of one or more ingredients capable of modifying physiological e.g. immunological functions of the organism it is administered to, or of organisms living in or on the organism. The term includes, but is not restricted to antibiotics or antiparasitics, as well as other constituents commonly used to achieve certain other objectives like, but not limited to, processing traits, sterility, stability, feasibility to administer the composition via enteral or parenteral routes such as oral, intranasal, intravenous, intramuscular, subcutaneous, intradermat or other suitable route, tolerance after administration, controlled release properties,

### Disclosure of the invention

The solution to the above technical problem is achieved by the description and the embodiments characterized in the claims.
The long lasting need in the art has been overcome for a live BVDV (bovine viral diarrhea virus) of defined sequence and specificity correlated to virulence which can be used to generate specific attenuated BVDV for use e.g. in a vaccine. A method to generate infectious clones and infectious BVDV particles derived thereof of defined genetic identity which at the same time have the pathogenicity closely resembling the wild-type virus is provided. Furthermore, an infectious type 2 clone and infectious type 2 BVDV particles derived thereof is disclosed. Thirdly, having generated live infectious BVDV particles of defined sequence, a method to generate attenuated BVDV particles with genetic identity which may be attenuated by modification at only one defined genetic marker site is also disclosed. The disclosure allows to generate a causal link between genome modification and attenuation, which is essential in order to understand the functional mechanism of the attenuation and therefore helpful to assess the quality in use as a vaccine.

In a first important embodiment, the current disclosure relates to a DNA molecule containing a nucleotide sequence complementary to a BVDV RNA, wherein said RNA, when introduced into susceptible host cells, induces the generation of infectious BVDV particles
a) with the capability to induce viraemia and leukopenia in calves for a period of at least one day and at least one of the following clinical symptoms of the group comprising diarrhea and/or pyrexia lasting at least one day when infected with a dose of 6x10⁶TClD₅₀.
b) with authentical virulence as defined *supra* as compared to a wild-type BVDV isolate from which such DNA molecule has been derived; and/or
c) which are, when BVDV naive calves are infected at a dose of 6x10⁸-TCID₅₀ with such particles, lethal for at least 30 % of such calves within a period of 21 days; and/or
d) with a virulence of not less than 90 % of BVDV particles comprising an RNA with a sequence complementary to SEQ ID NO, 1; and/or
e) comprising a sequence complementary to SEQ ID NO. 1.

Said dose of 6x10⁸ TCID₆₀ of step a) is preferably administered as 2x10⁶ i.m. (gluteal muscle). 2x10⁸ intranaseally, and 2x10⁸ subcutaneously (over scapula) to obtain a total dose of 6x10⁸. Said clinical symptoms of step a) preferably should be observed in at least two thirds of all infected animals. Said leukopenia of step a) preferably shall be at least a 35% reduction below baseline on at least two consecutive days, wherein "baseline" relates to the average values of all animals 10 days before infection. Diarrhea is a typical symptom of infection with BVDV.

Preferably, in a DNA molecule as described *supra* the pyrexia of step a) is at least 40°C.

In a second important embodiment the current disclosure relates to an infectious BVDV clone, capable of serving as a template for transcription into an RNA, wherein said RNA. when introduced into susceptible host cells, induces the generation of infectious BVDV particles
f) with the capability to induce viraemia and leukopenia in calves for a period of at least one day and at least one of the following clinical symptoms of the group comprising diarrhea and/or pyrexia lasting at least one day when infected with a dose of 6x10⁶TCID₅₀; and/or
g) with authentical virulence as compared to a wild-type BVDV isolate from which such DNA molecule has been derived; and/or
h) which are, when BVDV naive calves aged from 3 to 6 months are infected at a dose of 6x10⁸TCID₅₀ with such particles, lethal for at least 30 % of such calves within a period of 21 days after infection; and/or
I) with a virulence of not less than 90 % of BVDV particles comprising an RNA with a sequence complementary to SEQ ID NO. 1; and/or
j) comprising a sequence complementary to SEQ ID NO. 1.

Said dose of 6x10⁸TCID₅₀ of step f) is preferably administered as 2x10⁶ i.m. (gluteal muscle), 2x10⁸ intranaseally, and 2x10⁸ subcutaneously (over scapula) to obtain a total dose of 6x10⁸. Said clinical symptoms of step a) preferably should be observed in at least two thirds of all infected animals. Said leukopenia of step f) preferably shall be at least a 35% reduction below baseline on at least two consecutive days, wherein "baseline" relates to the average values of all animals 10 days before infection.

Said infectious BVDV clone preferably is a type 1 or type 2 clone.

As it is important that said infectious BVDV clone is of authentical virulence, the virus that serves as the origin for constructing such clone is preferably obtained directly from a field isolate or retransferred to animals and subsequently reisolated from the animal with the strongest clinical symptoms and subsequently passaged no more than twice in cell culture, preferably once or not at all. The example (example 1) exemplifies this. The example demonstrates the cDNA-cloning of virus NY93/C which is, after several cell culture passages, retransferred into a bovine animal, reisolated and used for RNA preparation and cDNA cloning after not more than two cell culture passages of the reisolated virus.

Another important embodiment of the current disclosure is a BVDV particle generated by transcription using the DNA molecule or the BVDV clone as described herein, the transfection of suitable cells or cell lines with said RNA and the collection of the resulting BVDV particles produced by said cells. Yet another embodiment is a BVDV particle generated by cloning the DNA molecule or the BVDV clone as described herein into the genome of a suitable DNA virus, such DNA viruses being known to the artisan, followed by infection of suitable cells resulting In generation of BVDV particles produced by said cells. Preferably also, the DNA or infectious clone as described herein may be transfected into suitable cells which then produce the RNA as disclosed for classical swine fever virus (CSFV) by von Gennip et. al. (1999) for cells which stably express T7 Polymerase, Also preferably the DNA or infectious clone as described herein may be expressed under control of an eukaryotic promotor in eukaryotic cells leading to the generation of infectious BVDV particles being able to be secreted from the cell (as exemplified by V- Racaniello and D. Baltimore for poliovirus (1981)).
A highly important embodiment of the current disclosure is an infectious BVDV type 2 clone.
Preferably, said infectious BVDV type 2 clone, capable of serving as a template for transcription into an RNA, wherein said RNA, when introduced into susceptible host cells, induces the generation of infectious BVDV particles
k) with the capability to induce viraemia and leukopenia in calves for a period of at least 1 day and at least one of the following clinical symptoms of the group comprising diarrhea and/or pyrexia lasting at least one day when infected with a dose of 6×10⁸ClD₅₀: and/or
l) with authentical virulence as compared to a wild-type BVDV isolate from which such DNA molecule has been derived; and/or
m) which are, when BVDV naive calves aged from 3 to 6 months are infected at a dose of 6x10⁶TCID₅₀ with such particles, lethal for at least 30 % of such calves within a period of 21 days after infection; and/or
n) with a virulence of not less than 90 % of BVDV particles comprising an RNA with a sequence complementary to SEO ID NO. 1: and/or
o) comprising a sequence complementary to SEQ IP NO. 1.
Preferably, the current disclosure relates to a BVDV type 2 clone obtainable by a method characterized by the following steps:
aaa) a wild-type BVDV type 2 strain is isolated;
bbb) said wild-type BVDV type 2 strain is passaged in cell-culture;
ccc) said cell culture-passaged BVDV type 2 strain is used to infect bovine animals and a BVDV strain is re-isolated from the most severely infected animal;
ddd) said re-isolated BVDV type 2 strain is passaged no more than twice, preferably once, in cell culture;
eee) said re-isolated BVDV type 2 strain is reverse-transcribed and cloned resulting in a full-length cDNA clone, preferably the 5' and 3' ends are cloned using the RACE-technology.

Said infectious DNA clone may then be transcribed into RNA under appropriate conditions, said RNA is introduced into appropriate cells or cell lines and the resulting BVDV type 2 particle is collected.
Such a clone is exemplified in the non-limiting example 1 and characterized by the cDNA sequence SEQ ID NO. 1. Thus, a preferred embodiment relates to an infectious BVDV type 2 clone as disclosed herein as characterized by the DNA sequence of SEQ ID NO. 1 or a fragment, functional variant, variant based on the degenerative nucleic acid code, fusion molecule or a chemical derivative thereof. A non-limiting example is provided in example 1.
The current disclosure further relates to a BVDV type 2 particle generated by *in vitro* transcription of the BVPV clone as described herein into RNA, the transfection of suitable cells or cell lines with said RNA and the collection of the resulting BVDV particles produced by said cells. Preferably also, the DNA or infectious clone as described herein may be transfected into suitable cells which then produce the RNA as disclosed for classical swine fever virus (CSFV) by van Gennip et. al. (1999) for cells which stably express T7 Polymerase. Also preferably the DNA or infectious clone as disclosed herein may be expressed under control of an eukaryotic promotor in eukaryotic cells leading to the generation of infectious BVDV particles being able to be secreted from the cell (as exemplified by V. Racaniello and D. Baltimore for poliovirus (1981)).
Another highly important aspect described herein is a DNA molecule containing a nucleotide sequence complementary to a full-length BVDV type 2 RNA. Preferably, said DNA molecule is characterized by the sequence SEQ ID NO, 1. Thus, the invention further relates to a DNA molecule as disclosed herein as characterized by SEQ ID No. 1 or a fragment, functional variant, variant based on the degenerative nucleic acid code, fusion molecule or a chemical derivative thereof. A non-limiting example is provided in example 1.
Most preferably, the current disclosure relates to a DNA molecule as disclosed herein, consisting of the sequence as characterized by SEQ ID No. 1.

The current disclosure further relates to a RNA molecule complementary to the DNA molecule as described *supra,* or to the BVDV clone as described *supra.*
The current disclosure also relates to a RNA molecule obtainable by transcription of the DNA molecule as described *supra,* or the BVDV clone as described *supra.*

Another important aspect of the current disclosure is a method for the production of an infectious BVDV clone from a wild-type BVDV isolate, said infectious BVDV clone being complementary to a RNA having authentical virulence as compared to said wild-type isolate, comprising the steps of
p) Isolating viral particles from an infected animal;
   preferably passaging not more than twice on suitable cell culture cells;
q) preparing RNA from the viral particles;
r) generating full-length complementary DNA after reverse transcription of the RNA; wherein the reverse transcription includes a step at elevated temperatures sufficient to break or reduce secondary structures of the RNA, and the use of a thermostable enzyme for this step, said enzyme being active at these elevated temperatures;
s) incorporating the complementary DNA (cDNA) into a plasmid vector or into a DNA virus capable of directing the transcription of BVDV cDNA into RNA upon infection of suitable cells,
Said viral particles preferably are isolated during viremia (step k)). The full length complementary DNA (cDNA) of step m) preferably may be generated by assembling overlapping partial cDNA fragments (see also example 1).

Another preferred embodiment relates to a method for the production of an infections BVDV clone from a wild-type BVDV isolate, said infectious BVDV clone being complementary to a RNA having authentical virulence as compared to said wild-type Isolate, comprising the steps of
ppp) isolating RNA from cells of an infected animal during viraemia or optionally after killing of said animal from its organ(s);
qqq) generating full-length complementary BVDV DNA which preferably is assembled from DNA fragments after reverse transcription of the RNA; wherein the reverse transcription includes a step at elevated temperatures sufficient to break or reduce secondary structures of the RNA, and the use of a thermostable enzyme for this step, said enzyme being active at these elevated temperatures;
rrr) incorporating the complementary DNA (cDNA) into a plasmid vector or into a DNA virus capable of directing the transcription of BVDV cDNA into RNA upon infection of suitable cells.

Suitable cells for cell culture are Madin-Darby bovine kidney (MDBK) cells, RD (bovine testicular) cells or bovine Turbinat (BT) cells. Further suitable cells are known to the person skilled in the art.

The infectious clone produced by the method as disclosed herein is a type 1 clone or preferably a type 2 clone.

Another Important aspect described hereinis a method for the production of an infectious BVDV clone from a wild-type BVDV isolate, said infectious BVDV clone being complementary to a RNA having a virulence of not less than 90 % of said wild-type isolate, comprising the steps of
t) isolating viral particles from an infected animal;
u) passaging not more than twice in suitable cell culture cells; preferably once or not at all:
v) preparing RNA from the viral particles;
w) generating full-length complementary DNA after reverse transcription of the RNA; wherein the reverse transcription includes a step at elevated temperatures sufficient to break or reduce secondary structures of the RNA, and the use of a thermostable enzyme for this step, said enzyme being active at these elevated temperatures;
x) incorporating the complementary DNA (cDNA) into a plasmid vector or into a DNA virus capable of directing the transcription of BVDV cDNA into RNA upon infection of suitable cells.
Said viral particles preferably are isolated during viremia (step t)). The full length complementary DNA (cDNA) of step x) preferably may be generated by assembling overlapping partial cDNA fragments (see also example 1).

There was a particular difficulty in the art to clone the 5 and 3' region of an infectious BVDV. A method was developed to obtain authentical 5' and 3' regions. Surprisingly, this was possible by applying the RACE-technology. However, modification of this technique led to the surprising and unexpacted generation of BVDV clones of authentic virulence. Preferably, in the method as disclosed herein, the 5' end of the RNA is generated using RACE. Surprisingly, only by applying the RACE technology in conjunction with a thermostable polymerase it was possible to dissolve the secondary structure of the genome successfully.
Standard molecular biology methods are known to the skilled person and can also be found e.g. in Sambrook et al.(1989) Molecular Cloning: A Laboratory Manual, 2^{nd} ed., Cold Spring Harbor Laboratory Press, Cold Spring Harbor, New York and Bertram. S. and Gassen, H,G. Gentechnische Methoden, G. Fischer Verlag, Stuttgart. New York, 1991).
Preferably, the current disclosure relates to a method wherein RACE is carried out with a thermostable polymerase allowing reaction temperatures of at least 48°C, preferably 50-55°C, preferably also 56-60°C.

Having disclosed live infectious BVDV particles of defined sequence, a method to generate attenuated BVDV particles with a defined genetic identity which preferably are attenuated at only one defined genetic marker site is disclosed. This surprisingly allows the simple determination of revertants or the successful attenuation as only the presence of the genetic marker site needs to be determined by molecular biology methods known to the artisan. XIKE-B and XIKE-C of example 1 are non-limiting examples for such attenuated BVDV particles of defined sequence.

Another important aspect described herein is a method of BVD virus attenuation by introducing one or more mutations into the DNA molecule as described *supra* or the infectious BVDV clone as described *supra,* wherein said mutation or mutations lead to or Increase an attenuated phenotype of the recovered BVD virus.
Yet another important aspect described hereinis a method of attenuation of a BVDV strain, comprising the steps of
y) introducing one or more mutations into the DNA molecule as described *supra,* or into the infectious BVDV clone as described *supra;*
z) introducing the mutated DNA into susceptible host cells wherein said DNA is transcribed into RNA or introducing an RNA transcribed from said DNA into said cells; and
aa)collecting viral particles produced by these cells;
wherein said mutation or mutations results in attenuation.
A preferred aspect disclosed herein is a method of attenuation as described *supra,* wherein the mutation or mutations is a nucleotide substitution, deletion, insertion, addition, or combination thereof.
As disclosed herein, "mutation" means the replacement of a nucleotide by another (e.g. C for a T) a so-called "substitution" or any other mutation such as "deletion" or "insertion". "Deletion" means the removal of one or several nucleotides or amino acids.
As these infectious BVDV clones as disclosed herein are viruses of authentic virulence closely resembling wild-type viruses and at the same time having a defined genotype, said virus must be used as a positive control in animal experiments. Said infectious clones are excellent tools for generating specifically attenuated BVDV clones to be used for e.g. vaccination. The disclosure comprises BVDV clones wherein the RNase activity residing in glycoprotein E^{RNS} is inactivated. Preferably, said RNAse activity is inactivated by deletion and/or other mutation such as substitution. Preferably, said deletions and/or other mutations are located at the amino acids at position 295 to 307 and/or position 338 to 357.
Thus, a more preferred aspect described herein is a method of attenuation , wherein the mutation or mutations is in the glycoprotein E^{rns} and causes impaired or loss of function of the mutated protein.
A more preferred aspect as described herein is a method of attenuation, wherein the mutation consists of
bb) deletion of all or part of the glycoprotein E^{rns}; and/or
cc) deletion or substitution of histidine at position 300 of SEQ ID NO.1; and/or
dd) deletion or substitution of histidine at position 349 of SEQ ID NO. 1.
Most preferably, yet another important embodiment is a method for the attenuation of BVDV, comprising the mutation of a BVDV clone as disclosed herein at histidine positions 300 and/or 349 wherein the coding triplett is deleted or substituted.
Yet another important embodiment is a method for the attenuation of BVDV as disclosed herein, wherein the codon for histidine 300 is substituted by a codon for leucine.
Yet another important embodiment is a method for the attenuation of BVDV as disclosed herein, wherein the codon for histidine 349 is deleted.

Another Important embodiment described herein is an attenuated BVDV clone or BVDV strain obtainable by a method as disclosed herein.

Another important embodiment described herein is a vaccine comprising an attenuated BVDV clone or strain as disclosed herein, optionally in combination with a pharmaceutically acceptable carrier or excipient.

The current disclosure further relates to the use of a attenuated BVDV clone or strain as disclosed herein in the manufacture of a vaccine for the prophylaxis and treatment of BVDV infections.

Preferably, a vaccine as described herein refers to a vaccine as defined above, wherein one immunologically active component is a live BVDV, wherein the RNase activity in its protein E^{RNS} is inactivated. The term "live vaccine" refers to a vaccine comprising a particle capable of replication, in particular, a replication active viral component.

Preferably, a vaccine as disclosed herein comprises an attenuated BVD virus type 1 as disclosed herein combined with an attenuated BVD virus type 2 as disclosed herein or any other antigenetic group and a pharmaceutically acceptable carrier or excipient. Said vaccine may be administered as a combined vaccine. Most preferably, said attenuated BVD virus type 1 as disclosed herein may be administered first followed by an administration of an attenuated BVD virus type 2 as disclosed herein three to four weeks later.

Preferably, a vaccine as disclosed herein comprises an attenuated BVD virus type 1 as disclosed herein wherein the RNase activity in its protein E^{RNS} is inactivated, combined with an attenuated BVD virus type 2 as disclosed herein wherein the RNase activity in its protein E^{RNS} is inactivated, or any other antigenetlc group wherein the RNase activity in its protein E^{RNS} is inactivated, and a pharmaceutically acceptable carrier or excipient. Said vaccine may be administered as a combined vaccine. Most preferably, said attenuated BVD virus type 1 as described *supra* may be administered first followed by an administration of an attenuated BVD virus type 2 as described *supra* three to four weeks later.

The current disclosure preferably relates to a method of treating a BVDV-infected bovine animal with an attenuated BVDV as described supra, wherein the said attenuated BVDV or the vaccine composition as disclosed supra is administered to the bovine animal in need thereof at a suitable dosis as known to the skilled person and the reduction of BVDV symptoms such as viremia and leukopenia and/or pyrexia and/or diarrhea is monitored. Said treatment preferably may be repeated.
The following examples serve to further illustrate the present invention; but the same should not be construed as limiting the scope of the invention disclosed herein.

### Example 1

### MATERIALS AND METHODS

**Cells and viruses**, MDBK cells were obtained from the American Type Culture Collection (Rockville, Md.). Cells were grown in Dulbecco's modified Eagle's medium supplemented with 10% fetal calf serum (FCS; tested for the absence of pestivirus and antibodies against pestiviruses) and nonessential amino acids.
Bovine viral diarrhea strain New York '93 (field isolate VLS#399) was kindly provided by E. J. Dubovi (New York State College of Veterinary Medicine, Cornell University, Ithaca). The virus underwent one animal passage and was termed "New York '93/C" thereafter.

**Infection of cells, immunofluorescence assay and virus peroxidase assay.** Since pestiviruses are highly associated with their host cells, lysates of infected cells were used for reinfection of culture cells. Lysates were prepared by freezing and thawing cells 3 to 5 days after infection and were stored at -70°C. Unless indicated otherwise in the text, a multiplicity of infection (m.o.l.) of 0,1 was used for infection of culture cells.
For immunofluorescence and peroxidase assays, the infected cells were fixed with ice-cold acetone:methanol (1:1) for 15 min at -20°C, air dried and rehydrated with phosphate buffered saline (PBS). Cells were then incubated with a mixture of anti-BVDV monospecific antibodies directed against E2 (Weiland et al. 1989). After three washes with PBS, a fluorescein Isothiocyanate (FITC)-conjugated rabbit anti-mouse antibody (Dianova, Hamburg, Germany) was used for detecting bound antibodies in the immunofluorescence assays. For peroxidase assays, peroxidase-conjugated goat anti-mouse antibody (Dianova) was used as second antibody. After incubation for one hour at room temperature, cells were washed three times with PBS. Bound antibodies were detected with a solution composed of 50 mM sodium acetate buffer pH 5.0, 1 µM aminoethylcarbazole and 0,1 % H₂O₂.

**Northern (RNA) hybridization.** RNA was prepared 48 hours after infection by cesium density gradient centrifugation as described before (Rümenapf at al. 1989). Gel electrophoresis, radloactive labelling of the probe, hybridization, and posthybridization washes were done as described before (Riimenapf et al, 1989). A radioactively labelled PCR product (nucleotides 4301 to 5302) from strain New York 93/C was used as a probe.
**PCR and RT-PCR**, PCR was carried out either with *Tfl*-Polymerase (Promega, Mannheim, Germany) or with *Taq*-Polymerase (Appligene, Heidelberg, Germany) following the manufacturer's recommendations and using ca. 50-100 ng of DNA template and 25 pmol of each primer. The sequences of the primers used for amplification of the 5' end of the genome were upstream. T₂₅V primer (Display Systems Biotech, Copenhagert, Denmark); and downstream. CM79; CTCCATGTGCCATGTACAGCAGAG for the first round and CM86: CTCGTCCACATGGCATCTCGAGAC for the nested PCR. The primers used for amplification of the 3' end of the genome were upstream, CM46: GCACTGGTGTCACTCTGTTG for the first round and CM80: GAGAAGGCTGAGGGTGATGCTGATG for the nested PCR and downstream, nls-: GACTTTCCGCTTCTTTTTAGG. Reverse transcription PCR (RT-PCR) was was done with the Titan™ One Tube RT-PCR System (Boehringer Mannheim, Germany), using 2 µg of total RNA as a template and following the manufacturer's instructions. The primers for amplification of the E^{rns} coding region were upstream, CM28: GGAGAGAATATCACCCAGTG; and downstream, CM21: CTCCACTCCGCAGTATGGACTTGC.
The amplified RT-PCR products were purified by preparative agarose gel electrophoresis and elution with the Nucleotrap kit (Macherey-Nagel. Düren, Germany) as recommended by the manufacturer.

**Phosphorylation and ligation of DNA-oligonucleotides to the 3' ends of RNA.** For ligation of a DNA primer to the 3' end of the virus genome, the primer was phosphorylated. 10 µg of the oligonucleotide nls+: CCTAAAAAGAAGCGGAAAGTC were incubated with 5 units of T4 polynucleotide kinase (New England Biolabs, Schwalbach, Germany) in 30 µl kinase-mix (2 mM ATP, 50 mM Tris-HCl pH 7.5, 10 mM MgCl₂, 10 mM dlthiothreitol, 25 µg/ml bovine serum albumin) for 40 min at 37°C. The primer was passed through a sephadex G-15 spin column (Sambrook et al. 1989) and further purified by phenol/chloroform extraction and ethanol precipitation.
Ligation was carried out using 5 µg of total RNA prepared from Infected culture cells and 150 pmol of the phosphorylated oligonucleotide with 20 units of T4-RNA-Ligase (New England Biolabs, Schwalbach, Germany) in 50 µl of ligase-mix (50 mM Tris-HCl pH 7.8, 10 mM MgCl₂, 10 mM dithiothreitol, 1 mM ATP, 40% polyethylene glycol and 50 units of RNA guard (Amersham, Freiburg. Germany)) for 16 hours at 17°C. The product was purified by phenol/chloroform extraction and ethanol precipitation.

**Synthesis and tailing of single-stranded DNA.** Single-stranded (-) DNA from the 5' end of the virus genome was generated with displayThermo-RT reverse transcriptase (Display Systems Biotech, Copenhagen. Denmark) using 2 µg of total RNA from infected cells and 100 pmol of primer CM79 (see "PCR and RT-PCR"), and following the manufacturer's Instructions (reaction: 65 °C for 10 min, 42°C for 40 min, 65°C for 15 min). The DNA was purified by two sequential phenol/chloroform extractions and ethanol precipitations with ¼ vol of 10 M ammonium acetate (Schaefer 1995).
A poly-dA tail was added to the first cDNA strand with Terminal deoxynucleotidyl Transferase (TdT) (Roche Molecular Biochemicals, Mannheim, Germany) using 50% of the "first strand" product, 50 units terminal transferase, 6,25 µM dATP and 1,5 mM CoCl₂ in 50 µl of TdT buffer as recommended by the manufacturer. After incubation at 37°C for 30 min, the product was purified by phenol/chloroform extraction and ethanol precipitation.

**Construction of a cDNA library and nucleotide sequencing.** Synthesis of cDNA, cloning and library screening were generally carried out as described previously (Meyers et al, 1991). cDNA synthesis was primed with oligos BVD13, BVD14 and BVD15 (Meyers et al. 1991) as well as with B22.1R (GTTGACATGGCATTTTTCGTG). B12.1R (CCTCTTATACGTTCTCACAACG). BVD33 (GCATCCATCATXCCRTG^{A}T_{G}AT), N7-3-7 (CAAATCTCTGATCAGTTGTTCCAC), B23-RII (TTGCACACGGCAGGTCC), and B-3' (GTCCCCCGGGGGCTGTTAAGGGTTTTCCTAGTCCA). The probe used for screening the library was the *Xho*I/*Aat*tI insert of a cDNA clone from BVDV strain cp7 (GenBank accession no. U63479. Meyers et al. 1996b); hybridisation was carried out at 52°C.
Exonuclease III and nuclease S1 were used to establish deletion libraries of cDNA clones (Henikoff 1987). Nucleotide sequencing of double-stranded DNA was carried out with the BigDye Terminator Cycle Sequencing Kit (PE Applied Biosystems, Weitersfiadt, Germany). As a rule, both DNA strands of the cDNA clones were sequenced; overlaps between independent cDNA clones were sequenced on at least two clones. In total, about 47.000 nucleotides were analyzed which equals an overall coverage of - 3.8 for the entire genome. Sequence analysis and alignments were done with Genetics Computer Group software (Devereux et al. 1984).

**Construction of the full-length cDNA clone.** Restriction, cloning and other standard procedures were generally carried out as described elsewhere (Sambrook et al. 1989). Restriction and modifying enzymes were obtained from New England Biolabs (Schwalbach, Germany), Pharmacia (Freiburg. Germany), GibcoBRL (Eggenstein, Germany), and Boehringer Mannheim (Germany).
Five cDNA clones from the library were used for construction of the full length cDNA clone: plasmid C3/8 (nucleotides 35 to 2411), plasmid C5/11 (nucleotides 22 to 2400) plasmid 8/11 (nucleotides 3400 to 7814), plasmid 13/27 (nucleotides 4783 to 9910) and plasmid C4/24 (nucleotides 8658 to 12322). A fragment "RT-E2" reaching from nucleotide position 2144 to position 4447 was obtained by RT-PCR with primers CM29 (GATGTAGACACATGCGACAAGAACC) and CM51 (GCTTCCACTCTTATGCCTTG), using total RNA from MDBK cells infected with field isolate VLS#399 as a template. In the following description, plasmid restriction sites flanking the viral cDNA Inserts are underlined.
First, clone C3/8 was cut with Aatll and *Hind*III, and the cDNA insert was transferred to PACYC177 cut with the same enzymes. The resulting plasmid was named pKANE5. RT-PCR product "RT-E2" was inserted into the *NdelI Hind*lll sites of this plasmid after restriction with the same enzymes: the resulting plasmid was pKANE8. Then, the *Aat*lI fragment from clone C5/11 was transferred into the *Aat*lI site of pKANE8, yielding plasmid pKANE14. The 5' end of the recombinant cDNA clone was generated by PCR with primers CM87 (GCTCTAGACGGCCGTAATACGACTCACTATAGGTATACGAGATTAGCTAAAGAAC TCGTATATGGATTGGACGTCAAC) that introduces a T7 promoter sequence upstream of the first cDNA nucleotide, and CM79 (see "PCR and RT-PCR"); plasmid C5/11 was used as the PCR template. The PCR product was ligated into the Xbal and BsrGl sites of cDNA clone C5/9, resulting in plasmid pKANE22. Later it was found that oligo CM87 contained a false nucleotide, and pKANE22 was repaired by PCR with oligos CM88 (GACGGCCGTAATACGACTCACTATAGTATACG) and CM79. The PCR product was treated with *E*. *coli* DNA-Polymerase I (Klenow fragment) to produce blunt ends and then restricted with *B*s*r*GI. It was cloned into the Spel^{blunt} / *Bsr*GI sites of pKANE22. resulting in plasmid pKANE22A.
The insert of cDNA clone 8/11 was cut with *Xho*I and *Bam*HI and cloned into pACYC177 cut with the same enzymes: the resulting plasmid was named pKANE6. The *Avr*II / *Bam*HI fragment of cDNA clone 13/27 was transferred to pKANE6, yielding plasmid pKANE15. Then, the *Eco*RV / *Mfe*I fragment from pKANE14 was inserted into pKANE15 digested with the same enzymes. The resulting plasmid was pKANE21. pKANE21 was digested with *Sac*II and *Eco*RV, and a corresponding fragment from pKANE14 was cloned into these sites, leading to plasmid pKANE24. Then the *Sac*II/*Sac*II fragment from pKANE 22A was cloned into pKANE24 cut with the same enzyme. The resulting plasmid was pKANE28AII.
The 3' end of the genome was generated by PCR with primers B2-11500 (CCTAACCATGATATATGCCTTCTG) and CM81 (CGGAATTCGCCCGGGCTGTTAGAGGTCTTCCCTAGT) which adds an *Srf*I, site to the 3' end of the genome. The PCR product was cut with *Bam*Hl and *Eco*RI and cloned into pACYC177, resulting in plasmid pKANE17. Then, the *Sac*l/*Kpn*2I fragment of cDNA clone C4/24 was transferred to pKANE17; the plasmid was called pKANE20. The *Stu*l / *Eco*Rl fragment was excised from pKANE20 and cloned into plasmid pKANE21 which was digested with *Eco*RI I and partially digested with Stul. The resulting plasmid was pKANE23, Finally, the *Xba*I / *Psh*AI fragment from pKANE28AII was inserted into plasmid pKANE23 cut with the same enzymes, leading to the full-length cDNA clone pKANE40.

**Site-directed mutagenesis.** All mutants were generated by PCR using the QuikChange site-directed mutagenesis kit (Stratagene, Amsterdam. Netherlands) following the manufacturer's instructions. The plasmid used for introducing mutations into the region coding for E^{rns} was C5/9, a clone obtained from the initial cPNA library (nucleotides 50 to 2411). Oligonucleotides for generating mutant H"346"Δ were CM126 (GAGTGGAATAAAGGTTGGTGTAAC) and CM 127 (GTTACACCAACCTTTATTCCACTC), oligos for mutant H"297"L were CM128 (AACAGGAGTCTATTAGGAATTTGGCCA) and CM129 (TGGCCAAATTCCTAATAGACTCCTGTT). The presence of the desired mutations and the absence of second site mutations were verified by nucleotide sequencing.

### in vitro transcription and RNA transfection

Transcription of RNA and transfection of MDBK cells were done essentially as described before (Meyers et al. 1996a). Briefly, 2 ug of the respective cDNA construct was linearized with Srfl and purified by phenol extraction and ethanol precipitation. Transcription with T7 RNA polymerase (NEB, Schwalbach, Germany) was carried out in a total volume of 50µl transcription mix (40mM Tris-HCl, pH 7.5; 6mM MgCl₂; 2mM spermidine; 10mM NaCl; 0.5 mM of each ATP. GTP, CTP and UTP; 10 mM dithiothreitol: 100ug/ml of bovine serum albumine) with 50 units of T7 RNA polymerase in the presence of 15 units RNAguard (Pharmacia, Freiburg, Germany). After incubation at 37°C for 1h the reaction mixture was passed through a Sephadex G-50 spun column and further purified by phenol extraction and ethanol precipitation.
If not specified otherwise, transfection was done with a suspension of ca. 3x10⁶ MDBK cells and about 0.5 µg of *in vitro* transcribed RNA bound to DEAE-dextran (Pharmacia, Freiburg, Germany). The RNA/DEAE-dextran complex was established by mixing RNA dissolved in 100 µl HBSS (5 g of Hepes, 8 g of NaCl, 0.37 g of KCl, 0.125 g of Na₂HPO₄2H₂O and 1 g of dextrose per Liter, pH 7.05) with 100 µl DEAE-dextran (1 mg/ml in HBSS) and incubation for 30 minutes on ice. Pelleted cells were washed once with DMEM without FCS, centrifuged and then resuspended in the RNA/DEAE-dextran mixture, After 30 minutes incubation at 37°C, 20 µl dimethyl sulfoxide was added and the mixture incubated for 2 minutes at room temperature. After addition of 2 ml HBSS, cells were pelleted and washed once with HBSS and once with medium without FCS. Cells were resuspended in DMEM with FCS and seeded In a 10.0-cm-diameter dish. 48h to 72h post transfection cells were split and seeded as appropriate for subsequent analyses. Electroporation was used for determination of the specific infectivity of RNA, 3x10⁶ MDBK cells in 0.5 ml of phosphate buffered saline (PBS) without magnesium and calcium were mixed with appropriate amounts of RNA and transferred into a 2 mm electroporation cuvette. Electroporation was done with one pulse of 960 µF. 180 Volt in a Hoefer PG 200 Progenetor II. Afterwards, the cells were seeded in 3,5 cm dishes and analyzed by immunofluorescence about 20 h later.

**Determination of RNAse activity.** MDBK cells were infected with the recombinant viruses and grown for 48 hours. Cells infected with the wild type virus served as a positive control, and uninfected cells were used as a negative control. Cell preparation and measurement of RNAse activity were carried out as described before (Meyers et al. 1999) with the exception that incubation of the probes at 37°C was 30 min instead of 1 hour because longer incubation resulted in considerable background activity in MDBK cells.

**Animal experiments.** Two animal experiments were carried out to test the recombinant viruses. In the first experiment, two groups of 3 flecked cattle female animals (8 to 10 months old) were inoculated intranasally with 10⁵TClD₅₀ per animal. In the second experiment, 6 male Holstein and Holstein-cross calves (7 to 10 weeks old) were infected intranasally with 5x10⁵TClD₅₀ per animal. In the challenge experiment, animals were inoculated with 5×10⁶TClD₅₀. All animals were tested free of BVDV specific antigen and antibody prior to infection. The different groups were housed in separate isolation units. Clinical parameters were recorded daily as indicated in the results section. Blood was taken from the vena jugularis extema at the time points indicated in the results section and was stabilized with Heparin (ca. 35 I.U./ml) unless it was used for the production of serum.
In order to determine the presence of virus in the blood, buffy coats were prepared from all blood samples. 5 ml ice cold lysis buffer were added to an aliquot of heparin stabilized blood (containing ca. 10⁷ leucocytes) and incubated on ice for 10 min, followed by centrifugation The pellet was washed once with lysis buffer and twice with PBS without Ca²⁺ and Mg²⁺ before it was resuspended in 2 ml PBS. MDBK cells seeded in 24-well plates were inoculated with 200 µl of the buffy coat preparations and incubated for 5 days. Viral antigen was detected by immunofluorescence microscopy with the BVDV E2 mAb mix (see above).
The presence of virus-neutralizing antibodies was tested in serum samples that had been inactivated by incubation at 56°C for 30 min. The sera were diluted in steps of 1:2 on 96 well microtitre plates and inoculated with a suspension of strain New York '93/C /100 TCID₅₀ per well) for 1 hour at 37°C. 10^{1.75} MDBK cells were added to each well and incubated for 5 days. Infection was analysed by immunofluorescence, calculated by the method of Kaerber (Mayr et al. 1974) and expressed as the 50 % endpoint dilution which neutralized approx. 100 TCID₅₀.
To detect virus in nasal discharge, nasal swabs were taken at the time points indicated in the results section, diluted in 2 ml of transport buffer (PBS supplemented with 5 % FCS, 100 I.U./ ml penicillin G, 0.1 mg / ml streptomycin and 2.5 µg / ml amphotericin B) and passed through a 0.2 µm filter, MDBK cells were inoculated in 24 well plates with 100 µl of these preparations and analysed by indirect immunofluorescence microscopy after 5 days.

### RESULTS

**Genome analysis.** The strain NY'93/C is the second BVDV type 2 genome that has been fully sequenced. Northern blot analysis showed that, contrary to strain 890 (Ridpath and Bolin 1995), the genome of NY'93/C contains no large insertions or deletions (data not shown). Nucleotide sequence analysis revealed that the genome is 12332 nucleotides long and contains one open reading frame encoding a polyprotein of 3913 amino acids.
The 5' untranslated region (position 1 to 365) was determined by RACE technology and was found to be identical with the New York '93 sequence published by Topliff and Kelling (1998) except for position 21. In contrast to other known type 2 genomes (Ridpath 1995: Topliff and Kelling 1998), strain NY'93/C has adenine at this position instead of thymin.

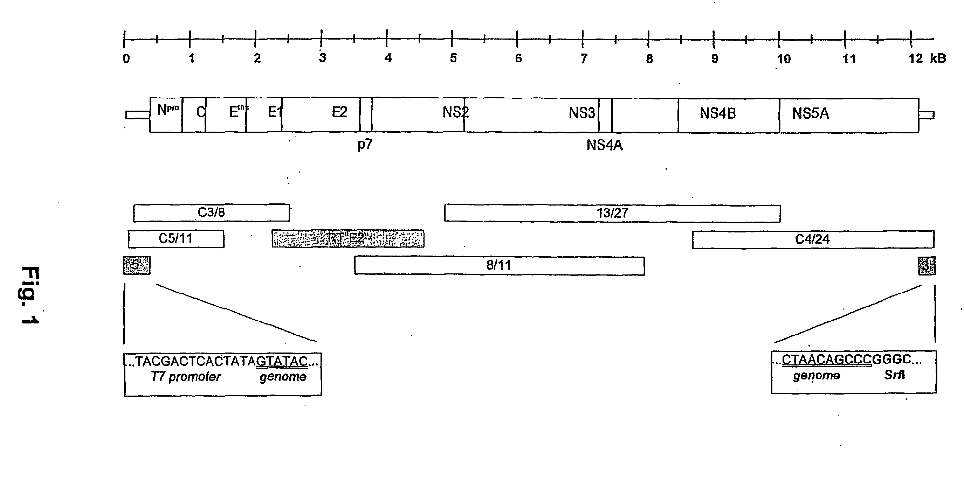

**Construction and analysis of an infectious cDNA clone for NY'93/C.** Although a number of infectious cDNA clones have been established for CSFV and BVDV type 1 (Mendez et al. 1998; Meyers et al. 1996a and 1996b; Moormann et al. 1996; Vassilev et al 1997; Kümmerer and Meyers 2000), this is the first report of an infectious clone from a BVDV type 2 strain. The clone was designed for runoff transcription with T7 RNA polymerase, resulting in a genome-like RNA without any heterologous additions.
The full-length clone was constituted from four cDNA plasmids selected from the initial phage library and one RT-PCR product encompassing the region between positions 2265 and 4301, At the 5' end, the sequence of the T7 promoter was added for *in vitro* transcription, and an Srtl site was added to the 3' end for plasmid linearization (Fig. 1). The full-length clone was named pKANE40A.

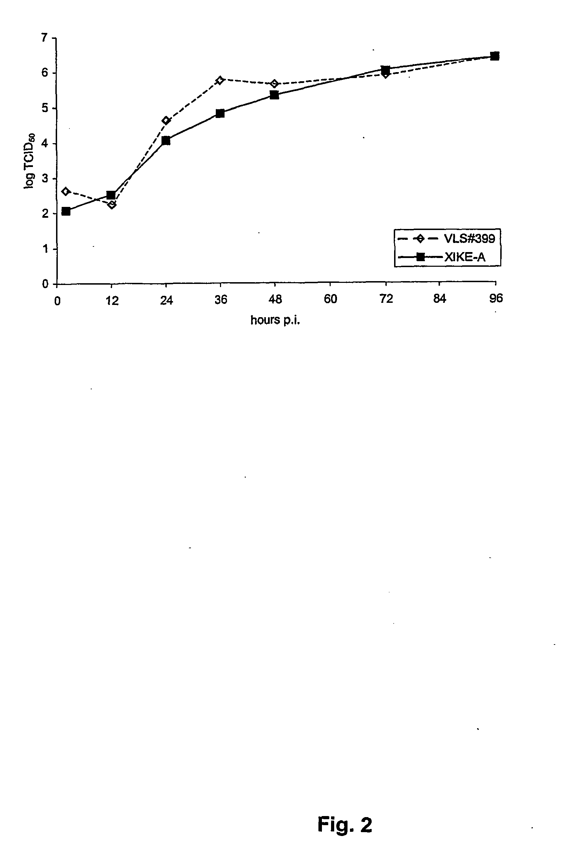

MDBK cells were transfected with RNA generated from the linearized pKANE40A template by *in vitro* transcription. A runoff transcript from plasmid pKANE28All which terminates 19 codons upstream of the NS5B coding region served as a negative control. Three days post transfection, BVDV-specific signals were detected after immunofluorescence staining in cells transfected with RNA from pKANE40A but not in the control. The virus generated from the infectious clone pKANE40A was termed XIKE-A, The transfected cells were passaged twice, and the stock of the second passage was used for all further experiments. The virus was analysed by RT-PCR sequencing, taking the nucleotide exchange from C to T at position 1630 as proof of the identity of XIKE-A.
The specific infectivity of the RNA derived from pKANE40A was determined in comparison to RNA prepared from cells infected with the wild type virus NY'93/C. To this end, the concentration of viral RNA in samples used for transfection of MDBK cells was measured in comparison with defined amounts of the *in vitro* transcribed RNA after Northern blotting and hybridization, using a phosphoimager. MDBK cells were transfected with similar amounts of both RNAs, and plaques were counted three days post transfection. On the average, the infectivity of RNA derived from pKANE40A was 4.32 x 10² pfu/µg, and the wild-type RNA yielded 4 x 10² pfu/µg.
The growth characteristics of the recombinant virus were analysed through a growth curve, using the original field isolate VLS#399 as a control in the same experiment (Fig. 2). MDBK cells were infected with an m.o.i. of 0.1, and samples were taken at seven time points from 2 hours to 96 hours post infection. The growth curve of the recombinant XIKE-A is somewhat smoother than that of VLS#399, but both viruses reach a titre of 10^{6,39} after 96 hours, XIKE-A was therefore deemed suitable for further experiments.

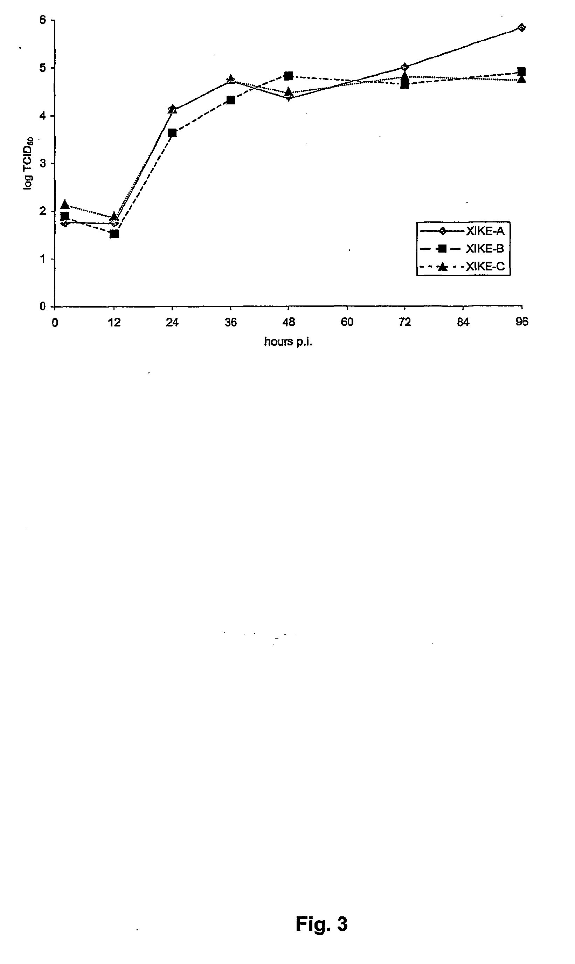

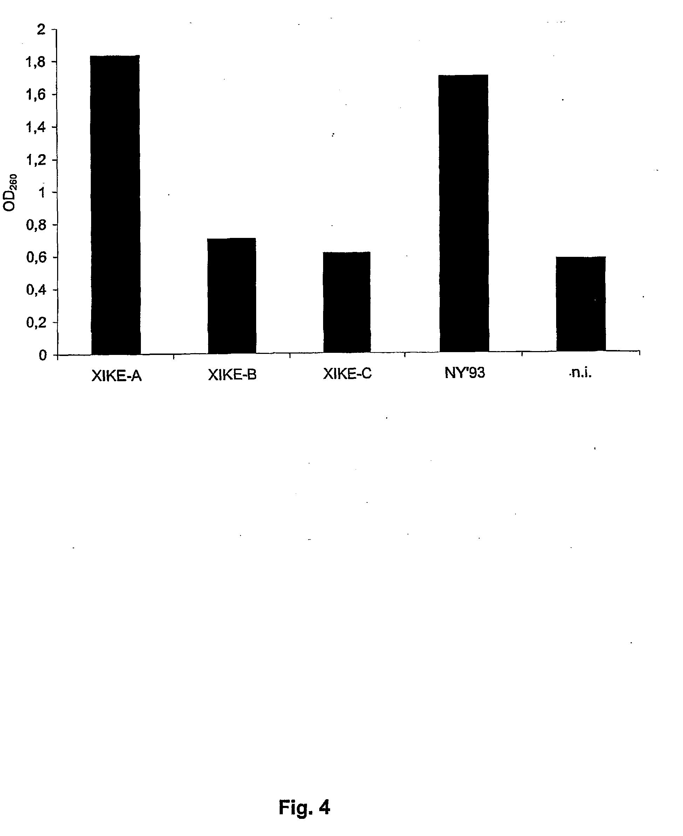

**Construction and analysis of E**^{**rns**} **mutants.** Previous experiments with CSFV (Meyers et al. 1999) had shown that the RNAse activity of the glycoprotein E^{rns} is destroyed by substitution of histidine 297 or 346 (the numbers represent the residue positions in CSFV strain Aifort/Tubingen) by leucine or lysin, or by deletion of codon "H346". The mutant viruses are viable, but clinically attenuated, In BVDV strain NY'931C, the two histidine residues are located at position 300 and 349, respectively. To test whether the effects of mutations at these positions would be similar to CSFV in a BVDV type 2 genome, two infectious clones were engineered with either a deletion of codon "H349" or a substitution of codon "H30D" by leucine. The resulting recombinant virus mutants were named XIKB-B (H349Δ) and XIKE-C (H300L).
Both mutants were stable in MDBK cells for at least five passages as determined by nucleotide sequencing of RT-PCR products encompassing the E^{rns} coding region. The growth characteristics of the two mutant viruses were compared with virus derived from the wild type infectious clone XIKE-A (Fig. 3).
The RNAse activity of XIKE-A, XIKE-B and XIKE-C was determined in crude cell extracts of cells infected with the same m.o,i. of either virus two days post infection. Aliquots of the preparations were tested for their ability to degrade poly(U); cells infected with the wild type strain NY'93/C served as a positive control, and uninfected cells were used as a negative control. After 30 min of incubation, the residual high molecular weight RNA was precipitated, and OD₂₆₀ measurement of the supernatants revealed the presence of small degraded RNA fragments (Meyers et al. 1999). High RNAse activity was found in the NY'93/C and XIKE-A samples whereas the two mutants XIKE-B and XIKE-C were in the same range as the negative control (Fig. 4).

**Animal experiment with XIKE-A and NY'93/C,** The purpose of the first animal experiment was to compare the virulence and pafihogenicity of the recombinant virus XIKE-A derived from the infectious cDNA clone with the wild type strain NY'931C. Two groups of three animals (8 to 9 months old) were each infected with 10⁵TCID₅₀ of either XIKE-A (animals #615, #377, #091) or NY'93/C (animals #275, #612, #1610). Each group was housed in a separate isolation unit, Body temperatures and clinical signs were recorded daily; blood samples were taken on days 0, 2 to 16 and 21 p.i, for leukocyte counts and detection of viremia. Sera from all calves were collected for detection of neutralizing antibodies against NY'93/C on days 0, 7, 14, 21, 29 and 35 p.l.. Nasal swabs for virus isolation were taken on day 0, 2 to 16 and 21 p.i.

**Table 1: Virus isolation from buffy coat preparations and nasal swabs of animals infected with New York '93/C or XIKE-A. + virus detected, - no virus detected, bac = bacteria,**

| Virus isolation from buffy coat preparations | | | | | | | Virus isolation from nasal swabs | | | | | |
|---|---|---|---|---|---|---|---|---|---|---|---|---|
| Days p.i | #275 | #612 | #1610 | #615 | #377 | #091 | #275 | #612 | #1610 | #615 | #377 | #091 |
| -26 | -- | -- | -- | -- | -- | -- | -- | -- | -- | -- | -- | -- |
| 0 | -- | -- | -- | -- | -- | -- | -- | -- | -- | -- | -- | -- |
| 2 | -- | -- | -- | -- | -- | -- | -- | -- | -- | -- | -- | -- |
| 3 | ++ | -- | -- | -+ | ++ | ++ | -- | -- | -- | -- | -- | -- |
| 4 | -+ | ++ | ++ | ++ | ++ | -+ | -- | -- | -- | -- | -- | -- |
| 5 | ++ | ++ | +- | ++ | ++ | ++ | -- | -- | -- | -- | -- | -- |
| 6 | ++ | ++ | ++ | ++ | ++ | ++ | -- | -- | -- | -- | -- | -- |
| 7 | ++ | -+ | ++ | ++ | ++ | ++ | -- | -- | -- | -- | -- | -- |
| 8 | ++ | -- | -- | ++ | ++ | ++ | -- | -- | -- | -- | -- | -- |
| 9 | -- | -- | -- | -- | ++ | -- | ++ | +- | -- | ++ | ++ | ++ |
| 10 | -- | -- | -- | -- | -- | -- | bac | -- | -- | +- | bac | +- |
| 11 | ++ | -- | -- | -- | -- | -- | bac | -- | -- | -- | -- | -- |
| 12 | -- | -- | -- | -- | -- | -- | -- | -- | -- | -- | -- | -- |
| 13 | -- | -- | -- | -- | -- | ++ | -- | -- | -- | -- | -- | -- |
| 14 | -- | -- | -- | -- | -- | * | -- | -- | -- | -- | -- | * |
| 15 | -- | -- | -- | -- | -- | * | -- | -- | -- | -- | -- | * |
| 16 | -- | -- | -- | -- | -- | * | -- | -- | -- | -- | -- | * |
| 21 | -- | -- | -- | -- | -- | * | -- | -- | -- | -- | -- | * |
| total | 7 | 4 | 4 | 6 | 7 | 7 | 1 | 1 | 0 | 2 | 1 | 2 |
| ∅ | | 5 | | | 6,7 | | | 0,7 | | | 1,7 | |

| | | | | | | | | | | | | |
|---|---|---|---|---|---|---|---|---|---|---|---|---|
| *animal was euthanized on day 13 p.i. | | | | | | | | | | | | |

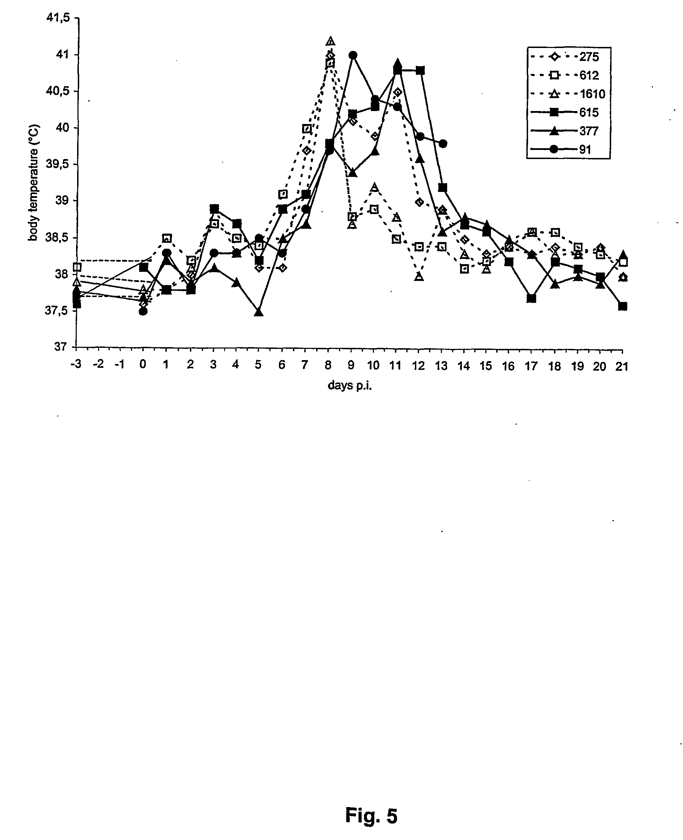

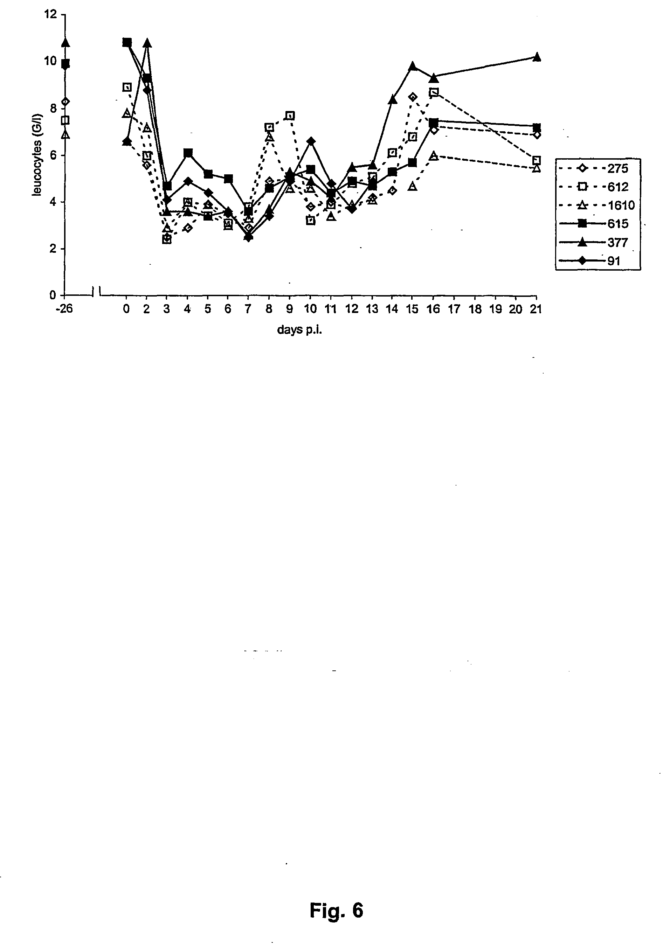

All animals in both groups developed fever (Fig. 5) and a broad spectrum of clinical signs including respiratory symptoms and gastrointestinal disorders. Animal #091 was killed on day 13 p.i. for welfare reasons. All calves in both groups showed leukopenia starting on day 3 p.i. and persisting for up to day 15 p.i. (Fig. 6). Virus was detected in buffy coat preparations from animals infected with NY'93/C for 5 days, and with XIKE-A for 7 days. Nasal shedding was found for 1 or 2 days (Table 1).
The identity of the viruses was checked by nucleotide sequencing of RT-PCR products from RNA prepared from buffy coat preparations from all animals. The entire E^{rns} coding region (positions 1140 to 1780) was sequenced and found to be identical with the known sequences of NY'93/C or XIKE-A, respectively. Neutralizing antibodies were found in the serum of all calves starting on day 14 p.i. (Table 2).

**Table 2: Neutralizing antibody titres determined in serum samples of all calves after experimental infection with New York '93/C or XIKE-A. Results are expressed as the reciprocal of the serum BVDV-specific neutralizing antibody titers against New York '93/C (10^{2,07} TCID₅₀).**

| **days p.i.** | **615** | **377** | **091** | **275** | **612** | **1610** |
|---|---|---|---|---|---|---|
| **-26** | <2 | <2 | <2 | <2 | <2 | <2 |
| **0** | <2 | <2 | <2 | <2 | <2 | <2 |
| **7** | <2 | <2 | <2 | <2 | <2 | <2 |
| **13/14** | 645 | 323 | 406 | 256 | 128 | 40 |
| **21** | 1024 | 1290 | * | 1290 | 512 | 51 |
| **29** | 2580 | 4096 | * | 813 | 2580 | 2580 |
| **35** | 3251 | 3251 | * | 8192 | 2580 | 5161 |

| | | | | | | |
|---|---|---|---|---|---|---|
| * animal were euthanized on day 13 | | | | | | |

The results of this study demonstrated that the recombinant virus XIKE-A is highly similar to the wild type virus NY'93/C with regard to both pathogenicity and an the induction of an immune response in the natural host. It is therefore plausbile to assume that any deviation from this clinical picture that might be observed in a virus mutant generated on the basis of the infectious clone pKANE40A would indeed be caused by the desired mutation.

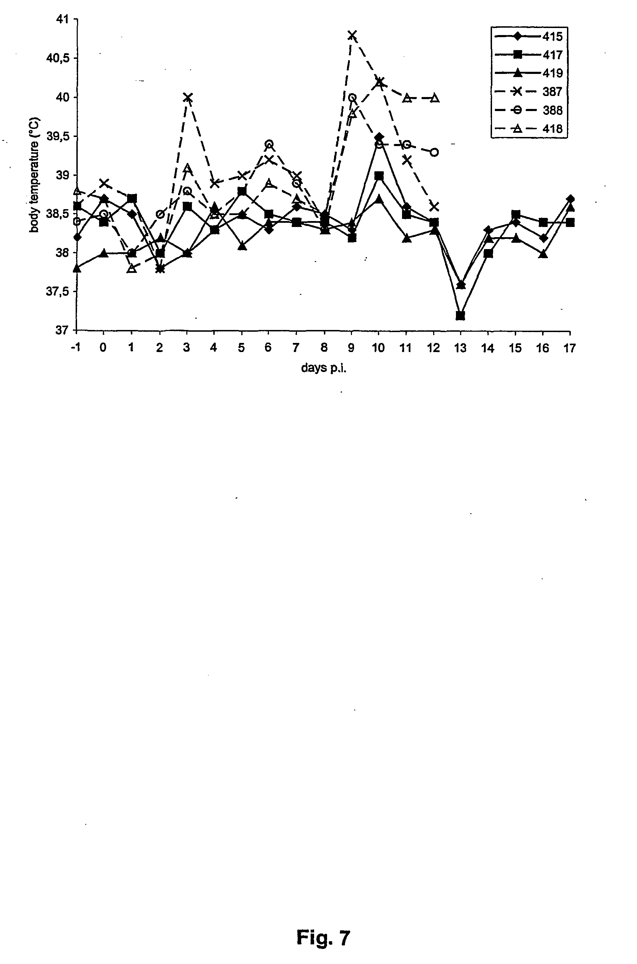

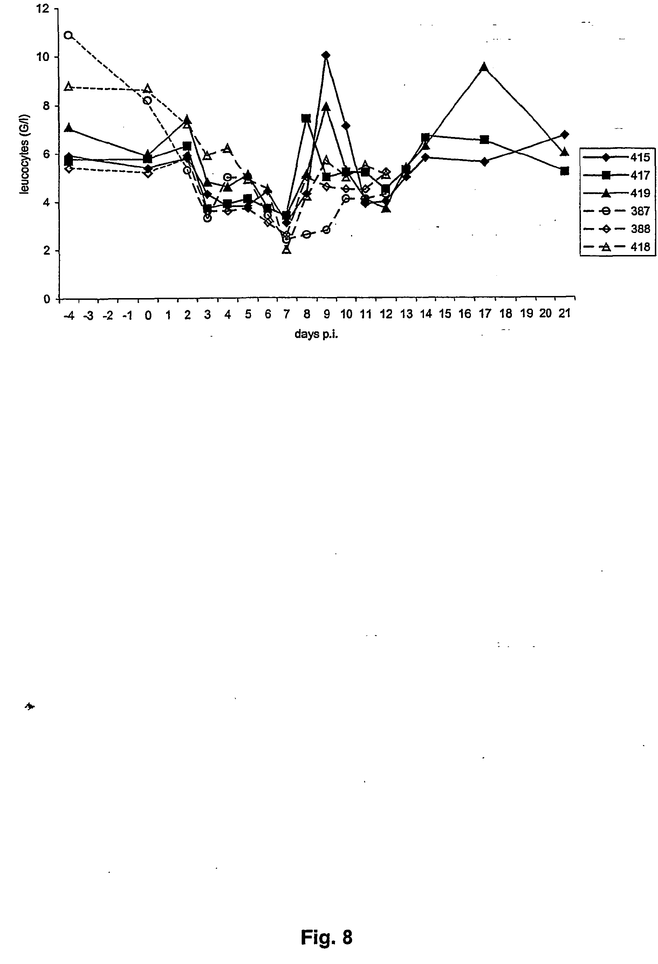

**Animal experiment with XIKE-B and XIKE-A.** In the second animal experiment, the clinical and immunological characteristics of the RNAse negative mutant XIKE-B were analysed in comparison with XIKE-A. The H349Δ mutant was given precedence over the H300L mutant to minimize the danger of a genomic reversion to wildtype.
Two groups of three calves (7 to 10 weeks old) each were inoculated with a dose of 5x10⁵TCID₅₀ of either XIKE-A (animals #387, #388, #418) or XIKE-B virus (animals #415, #417, #419). The groups were housed in separate isolation units. Rectal temperatures and clinical symptoms were monitored daily; nasal swabs and blood samples were taken on days -8, 0, 2 to 14, 17 and 21. Serum samples were collected on days 0, 8, 12 /14, 21, 28 and 38/40.
Nine to ten days post infection, the calves infected with XIKE-A developed fever for up to 3 days; in addition animal #387 had fever on day 3 p.l. (Fig. 7) that was accompanied by diarrhea and respiratory symptoms. Calf #388 showed convulsions. The group was euthanized for welfare reasons on day 12 p.i. in a state of marked depression and anorexia. None of the calves infected with XIKE-B had elevated body temperatures (Fig. 7). Only mild respiratory symptoms were observed for up to 6 days. Leucopenia was found in all animals; however, the decrease of leucocyte numbers was more pronounced in the calves infected with wild type XIKE-A than in the XIKE-B group (Fig. 8).
Virus was found in buffy coat preparations of all animals starting on day 4 p.i.; however, viremia was shorter for the E^{rns} mutant (∅ 4 days) than for the virus with wild type sequence (∅ 8 days). Nasal shedding of virus could be observed for up to 8 days (∅ 4,7) with XIKE-A animals, but for a maximum of 1 day (∅ 0,7) with XIKE-B animals (Table 3).

**Table 3: Virus isolation from buffy coat preparations and nasal swabs of animals infected with the recombinant virus XIKE-A (animals #387, #388 and #418) or the E^{rns} mutant XIKE-B (animals #415, #417 and #419). +virus detected, -no virus detected, *animals were euthanized on day 12 p.i.**

| Virus isolation from buffy coat preparations | | | | | | | Virus isolation from nasal swabs | | | | | |
|---|---|---|---|---|---|---|---|---|---|---|---|---|
| Days p.l. | #415 | #417 | #419 | #387 | #388 | #418 | #415 | #417 | #419 | #387 | #388 | #418 |
| -8 | -- | -- | -- | -- | -- | -- | -- | -- | -- | -- | -- | -- |
| 0 | -- | -- | -- | -- | -- | -- | -- | -- | -- | -- | -- | -- |
| 2 | -- | -- | -- | -- | -- | -- | -- | -- | -- | -- | -- | -- |
| 3 | -- | -- | -- | -- | -- | -- | -- | -- | -- | -- | -- | -- |
| 4 | +- | +- | -- | ++ | ++ | ++ | -- | -- | -- | -- | -- | -- |
| 5 | ++ | +- | +- | ++ | ++ | ++ | -- | -- | -- | -- | -- | +- |
| 6 | ++ | +- | ++ | ++ | ++ | ++ | -- | +- | -- | -- | -- | +- |
| 7 | ++ | ++ | ++ | ++ | ++ | ++ | -- | -- | +- | +- | -- | +- |
| 8 | -- | +- | -- | ++ | ++ | ++ | -- | -- | -- | -- | -- | +- |
| 9 | -- | -- | -- | ++ | +- | ++ | -- | -- | -- | ++ | ++ | +- |
| 10 | -- | -- | -- | ++ | +- | +- | -- | -- | -- | +- | +- | ++ |
| 11 | -- | -- | -- | ++ | +- | ++ | -- | -- | -- | +- | -- | +- |
| 12 | -- | -- | -- | -- | -- | -- | -- | -- | -- | -- | -- | ++ |
| 13 | -- | -- | -- | * | * | * | -- | -- | -- | * | * | * |
| 14 | -- | -- | -- | * | * | * | -- | -- | -- | * | * | * |
| - | | -- | -- | * | * | * | -- | -- | -- | * | * | * |
| 21 | | | | * | * | * | -- | -- | -- | * | * | * |
| total | 4 | 5 | 3 | 8 | 8 | 8 | 0 | 1 | 1 | 4 | 2 | 8 |
| ∅ | | 4 | | | 8 | | | 0,7 | | | 4,7 | |

Again, nucleotide sequencing of RT-PCR products encompassing the entire E^{rns} coding region was used for virus identification in buffy coat preparations. As expected, isolates from animals #387, #388 and #418 were wild type. A deletion of the "H349" codon was confirmed for animals #415, #417 and #419. Interestingly, an additional point mutation was found in RT-PCR products from two of these animals (#415 and #419): nucleotide position 1246 was changed from guanin to thymin, resulting in the amino acid substitution Q287H. Neutralizing antibodies were first detected on day 12 p.i. in the serum of the calves infected with XIKE-A, and on day 14 p.i. in the serum of calves infected with the E^{rns} mutant (Table 4).

**Table 4: Neutralizing antibody titres determined in serum samples of all calves after experimental infection with XIKE-A (wild type sequence) or XIKE-B (H346?). Results are expressed as the reciprocal of the serum BVDV-specific neutralizing antibody titers against New York '93/C (10^{1,7} TClD₅₀).**

| **days p.i.** | **387** | **388** | **418** | **415** | **417** | **419** |
|---|---|---|---|---|---|---|
| **0** | <2 | <2 | <2 | <2 | <2 | <2 |
| **8** | <2 | <2 | <2 | <2 | <2 | <2 |
| **12/14** | 20 | 8 | 128 | 51 | 203 | 64 |
| **21** | * | * | * | 512 | 1024 | 406 |
| **28** | * | * | * | 2048 | 1024 | 4096 |
| **38/40** | * | * | * | 8182 | 4096 | 4096 |

| | | | | | | |
|---|---|---|---|---|---|---|
| *animals were euthanized on day 12 | | | | | | |

### Example 2

### Experimental design

Twelve pregnant heifers were selected from a BVDV negative herd. The following group of 5 / 7 heifers were included in the trial:

| | No. | Inoculation | Virus |
|---|---|---|---|
| Group 1: | 5 | One i. n. administration, 3 ml in each nostril | XIKE-A |
| Group 2: | 5 | One i. n. administration, 3 ml in each nostril | NY-93 |

Heifers were moved to the experimental facilities 8 days before inoculations. Pregnancy status was confirmed after transport into the experimental facility. Heifers were between days 60 and 90 of gestation on the day of inoculation. Inoculation took place for all animals at one point of time with 2.5 x 10⁴ TCID₅₀/ml of the respective virus applied in 6ml tissue culture supernatant.

Heifers were monitored for the presence of clinical signs of BVDV infection including abortions during the observation period. The experiment was terminated 9 weeks after infection. Non-aborted cows were slaughtered, the uterus examined and collected. Foetal organ samples were collected during routine necropsy and examined for BVDV infection.

The presence of fetal infection was the main evaluation parameter, composed from the number of BVDV-related cow mortality, the number of BVDV-related abortions and the number of BVD positive fetuses at termination.

### Results:

### Group 1

| Animal No. | Conclusion |
|---|---|
| 526 | BVD abortion |
| 598 | BVD abortion |
| 615 | BVD abortion |
| 618 | BVD abortion |
| 626 | Heifer Died due to BVD |

### Group 2

| Animal No. | Conclusion |
|---|---|
| 184 | Heifer Died due to BVD |
| 203 | BVD abortion |
| 232 | Heifer Died due to BVD |
| 233 | Foetus BVD positiv (viremic) |
| 252 | BVD abortion |
| 267 | Heifer died due to BVD |
| 306 | BVD abortion |

### Example 3:

The study aimed to assess the efficacy of BVDV isolates against foetal infection. Efficacy of the NY93 infectious copy derivative BVDV recombinant (type II) with a deletion of the Rnase function in the E(RNS) protein XIKE-B (H349A) is investigated to prevent fetal infection after an heterologous type I challenge.
Between day 60 and 90 is the most sensitive period for fetal exposure to BVDV. Therefore in this trial heifers derived from BVDV-free farm (and confirmed seronegative for BVDV) have been immunized by a single exposure with XIKE B (i.m.). Thereafter heifers were inseminated and between day 60-90 animals, when animals are supposed to be highly sensitive to BVDV fetal infection, a challenge infection with a wild type field virus was performed. The intranasal route for challenge was chosen as this mimics the normal route of infection in the field best.

### Experimental design:

Heifers were selected from a BVDV negative herd. The heifers were tested serologically and virologically negative for BVDV. The following groups of heifers were included in the trial:

| Group | Treatment | Challenge | No. of heifers: | |
|---|---|---|---|---|
| | | BVDV | Vaccinated | Challenged |
| 1 | None | Type I | NA | 2 |
| 2 | Isolate XIKE-B | Type I | 10 | 4 |

Group 1 remained untreated in the herd of origin until challenge. Blood samples were collected post-vaccination for buffy coat preparation and serology.
Inseminations started 4 weeks after immunisation for all groups. Group 1 was transported to the experimental facility before challenge.
Heifers were challenged 4 months and 10 days after vaccination. At the day of inoculation, pregnancy status was between day 60 and 90 of pregnancy.

The prevention of foetal infection was the main evaluation parameter.

### Sequence of events and time schedule

| | |
|---|---|
| Immunisation | 10 days after transport to the animal facility |
| Insemination | In a period of 30 days, started approximately 4 weeks after Immunisation |
| Second transport to the challenge facility | At least 10 days before challenge |
| Challenge | Between day 60 to 90 of pregnancy |
| Observations | Continuous for about 2 months |
| Slaughter of animals and harvest of foetuses for virus isolation testing of fetal organ samples | About 2 months post-challenge |

### BVDV challenge viruses

The virus is grown in BVDV free medium as appropriate, aliquoted and frozen at -70 °C [±10 C].

| Type / designation:: | Type I / ncp KE#9 |
|---|---|
| Passage level: | 4 |
| Composition: | Isolate obtained from German field |
| Challenge dose: | 10⁵ per animal |
| Applied volume: | 6 ml per animal (3ml per nostril) |
| Inoculation route: | Intranasal |

### Vaccinations

The vaccination schedule is described in the Experimental Design Section.

| Description: | XIKE-B, live virus BVDV strain |
|---|---|
| Passage number: | 10 |
| Virus dose: | 10⁵ per animal |
| Applied vaccine volume: | 2 ml per animal |
| Application route: | Intramuscular (i. m.) |

### Results:

### Rectal temperatures

The temperature values were below 39°C in all but one cases, and no unusual fluctuations were seen during the observation period. Heifer N° 1249 (Group 1) had a temperature of 39.1 °C on 14 DPI (=days post infection) that returned to normal value on the next day.

### Leukocyte Counts

0 DPI values were considered as individual baseline for comparison. No lower limit of leukocyte counts was defined in the study protocol. However, a reduction of leukocyte counts by 40% or more, i.e., values reaching 60% of the baseline value (established on the day of challenge) or lower, was considered biologically significant.

Individual mean leukocyte counts are shown in Table 1 below.

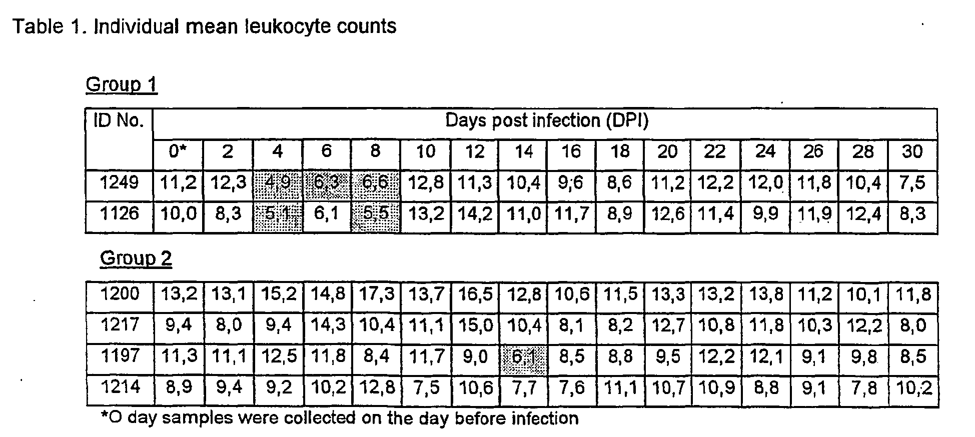

Baseline leukocyte counts were similar in all groups. While both heifers in Group 1 (infected with Type I strain) experienced a biologically significant reduction in leukocyte counts (values highlighted with grey colour) after the challenge (maximum drop noted 4 - 8 DPI), the corresponding vaccinated heifers (Group 2) had no remarkable falls in leukocytes. The only exception was heifer N° 1197 who showed a significant decrease on a single day, on Day 14 Pl. On the very next day, leukocyte count returned to what was considered normal (less than 40 % deviation from baseline).

### Virus isolation data

Methods applied for virus isolation investigations are detailed in previous examples. Virus isolation data from buffy coats (described as day post infection (=DPI) with vaccine candidate (XIKE B):

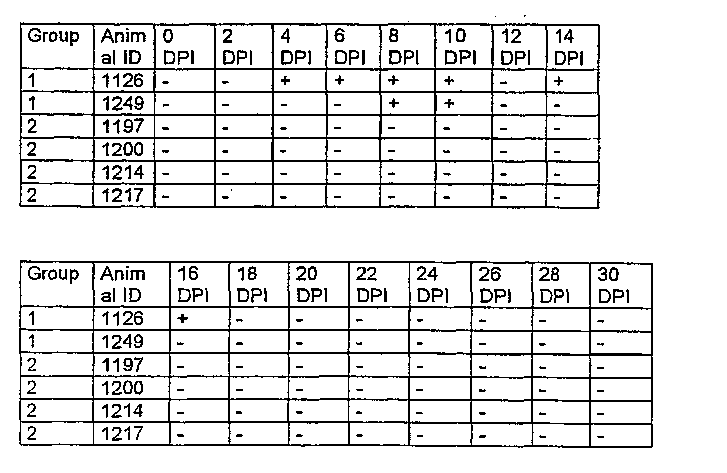

Virus isolation from fetal organs:

| Group | Animal ID | Mesenteric lymph nodes | Small intestine | Spleen | Thymus | kidney | Stemum bone marrow | cerebellum | placenta |
|---|---|---|---|---|---|---|---|---|---|
| 1 | 1126 | + | + | + | + | + | + | + | + |
| 1 | 1249 | + | + | + | + | + | + | + | + |
| 2 | 1197 | - | - | - | - | - | - | - | - |
| 2 | 1200 | - | - | - | - | - | - | - | - |
| 2 | 1214 | - | - | - | - | - | - | - | - |
| 2 | 1217 | - | - | - | - | - | - | - | - |

All heifers did not show any clinical symptomes typical for BVDV infection after vaccination with XIKE B. After challenge heifers of group 1 had on at least one day viremia, whereas in group 2 on no day after challenge viremia could be detected. All fetuses from heifers of group 1 were positive for BVDV (all of the following organs were positiv tested for BVDV by virus isolation (mesenteric lymph nodes; small intestine, spleen, thymus, kidney, sternum, bone marrow, cerebellum); the fetuses from from heifers of group 2 were all negative (in all tested organs consistently: mesenteric lymph nodes; small intestine, spleen, thymus, kidney, sternum, bone marrow, cerebellum) for BVDV.
Therefore infectious copy derived virus was attenuated successfully and the potential of the use as vaccine virus in order to prevent fetal infection was shown.

The XIKE B virus belongs antigenetically to the BVDV type II viruses and is effective in preventing fetal infection after challenge with an heterologous challenge virus belonging to the BVDV type I antigenic group.

### References

Baker, J.C. 1987. Bovine viral diarrhea virus: a review. J. Am. Vet. Med.Assoc. 190: 1449-1458.
Becher, P., Orlich, M., Kosmidou A., König, M., Baroth M., Thiel H.J., 1999. Genetic Diversity of Pestivirus: Identification of Novel Groups and Implications for Classification. Virology 262: 64-71.
Bolin, S. R., Littledike, E. T. and Ridpath, J. F. (1991): Serologic detection and practical consequences of antigenic diversity among bovine viral diarrhea viruses in a vaccinated herd. Am. J. Vet. Res. 52:1033-1037
Devereux, J., Haeberli, P. and Smithies, O. (1984): A comprehensive set of sequence analysis programs for the VAX. Nucleic Acids Res. 12:387-395
Flores, E. F., Gil, L. H., Botton, S. A., Weiblen, R., Ridpath, J. F., Kreutz, L. C., Pilati, C., Driemeyer, D., Moojen, V. and Wendelstein, A. C. (2000): Clinical, pathological and antigenic aspects of bovine viral diarrhea virus (BVDV) type 2 isolates identified in brazil. Vet. Microbiol. 77:175-83
Henikoff, S. (1987): Unidirectional digestion with exonuclease III in DNA sequence analysis. Methods Enzymol. 155:156-165
Hulst, M. M., Panoto, F. E., Hoekman, A., van Gennip, H. G. and Moormann, R. J. (1998): Inactivation of the RNase activity of glycoprotein E(rns) of classical swine fever virus results in a cytopathogenic virus. J. Virol. 72:151-7
Kümmerer, B. M., Tautz, N., Becher, P., Thiel, H. and Meyers, G. (2000): The genetic basis for cytopathogenicity of pestiviruses. Vet. Microbiol. 77:117-28.
Mayr, A., Bachmann, P. A., Bibrack, B. and Wittmann, G. (1974): Virologische Arbeitsmethoden Band I. Gustav Fischer Verlag, Stuttgart.
Mendez, E., Ruggli, N., Collett, M. S. and Rice, C. M. (1998): Infectious bovine viral diarrhea virus (strain NADL) RNA from stable cDNA clones: a cellular insert determines NS3 production and viral cytopathogenicity. J. Virol. 72:4737-45
Meyers, G., Tautz, N., Dubovi, E. J. and H.-J. Thiel (1991): Viral cytopathogenicity correlated with integration of ubiquitin-coding sequences. Virology 180:602-616
Meyers, G., Thiel, H. J. and Rumenapf, T. (1996a): Classical swine fever virus: recovery of infectious viruses from cDNA constructs and generation of recombinant cytopathogenic defective interfering particles. J. Virol. 70:1588-95
Meyers, G., Tautz, N., Becher, P., Thiel, H.-J., Kümmerer, B. M. (1996b): Recovery of cytopathogenic and noncytopathogenic bovine viral diarrhea viruses from cDNA constructs. J. Virol. 70:8606-13 (erratum J. Virol. 1997, 71:173)
Meyers, G., Saalmuller, A. and Büttner, M. (1999): Mutations abrogating the Rnase activity in glycoprotein Erns of the pestivirus classical swine fever firus lead to virus attenuation. J. Virol. 73:10224-10235
Moennig, V. and Liess, B. 1995. Pathogenesis of Intrauterine Infections with Bovine Viral Diarrhea Virus. 11: (3), 477-487.
Moennig, V. and Plagemann, J. 1992. The pestiviruses. Adv. Virus Res. 41: 53-91.
Moormann, R. J., van Gennip, H. G., Miedema, G. K., Hulst, M. M. and van Rijn, P. A. (1996): Infectious RNA transcribed from an engineered full-length cDNA template of the genome of a pestivirus. J. Virol. 70:763-70
Oirschot van, J.T., Bruschke, C.J.M., Rijn van, P.A., 1999. Vaccination of cattle against bovine viral diarrhoea. Veterinary Microbiology, 64: 169-183.
Racaniello, V. R. and Baltimore, D., 1981. Cloned poliovirus complementary DNA is infectious in mammalian cells. Science 214: 916-919.
Ridpath, J. F., Bolin, S. R. and Dubovi, E. J. (1994): Segregation of bovine viral diarrhea virus into genotypes. Virology 205:66-74
Ridpath, J. F. and Bolin, S. R. (1995): The genomic sequence of a virulent bovine viral diarrhea virus (BVDV) from the type 2 genotype: detection of a large genomic insertion in a noncytopathic BVDV. Virology 212:39-46
Ridpath, J. F., Neill, J. D. (2000): Detection and characterization of genetic recombination in cytopathic type 2 bovine viral diarrhea viruses. J. Virol. 74:8771-4.
Ridpath, J. F., Neill, J. D., Frey, M., Landgraf, J. G. (2000): Phylogenetic, antigenic and clinical characterization of type 2 BVDV from north America. Vet. Microbiol. 77:145-55
Rümenapf, T., Meyers, G., Stark, R. and Thiel, H.-J. (1989): Hog cholera virus characterization of specific antiserum and identification of cDNA clones. Virology 171:18-27
Sambrook, S. E., Fritsch, F. and Maniatis, T. (1989). Molecular cloning: a laboratory manual, 2^{nd} ed. Cold Spring Harbor Laboratory Press, Cold Spring Harbor, N.Y.
Schaefer, B. C. (1995): Revolutions in Rapid Amplification of cDNA Ends: New Strategies for Polymerase Chain Reaction Cloning of Full-Length cDNA Ends. Anal. Biochem. 227:255-273
Thiel, H.-J., Stark, R., Weiland, E., Rumenapf, T. and Meyers, G. (1991): Hog cholera virus: molecular composition of virions from a pestivirus. J. Virol. 65:4705-4712
Thiel, H.-J., Plagemann, G.W., & Moennig, V. 1996. The pestiviruses. In *Fields Virology,* eds. Fields, B.N., Knipe, D.M., & Howley, P.M. (Lippincott-Raven, Philadelphia), pp.1059-1073.
Tijssen, P., Pellerin, C., Lecomte, J (1996): Immunodominant E2 (gp53) sequences of highly virulent bovine viral diarrhea group II viruses indicate a close resemblance to a subgroup of border disease viruses. Virology 217:356-361
Topliff, C. L. and Kelling, C. L. (1998): Virulence markers in the 5' untranslated region of genotype 2 bovine viral diarrhea virus isolates. Virology 250:164-72
Van Gennip H. G., van Rijn, P. A., Widjojoatmodjo M. N., Moormann R.J. (1999): Recovery of infectious classical swine fever virus (CSFV) from full-length genomic cDNA clones by a swine kidney cell line expressing bacteriophage T7 RNA polymerase. J. Virol. Methods 78:117-128.
Vassilev, V. B., Collett, M. S. and Donis, R. O. (1997): Authentic and chimeric full-length genomic cDNA clones of bovine viral diarrhea virus that yield infectious transcripts. J. Virol. 71:471-8
Weiland, E., Thiel, H.-J., Hess, G. and Weiland, F. (1989): Development of monoclonal neutralizing antibodies against bovine viral diarrhea virus after pre-treatment of mice with normal bovine cells and cyclophosphamide. J. Virol. Methods 24:237-244

## Claims

1. Use of a recombinant BVDV type II virus that is encoded by a nucleotide sequence which is SEQ ID NO:1, but wherein histidine codon H349 within the E^{RNS} region is deleted, for the production of a medicament for the prevention of fetal infection with a heterologous virus belonging to the BVDV type I antigenic group.

## Patentansprüche

1. Verwendung eines rekombinanten BVDV Typ II-Virus, der durch eine Nucleotidsequenz, welche SEQ ID NO:1 ist, kodiert wird, worin jedoch das Histidin-Codon H349 innerhalb der E^{RNS}-Region deletiert ist, für die Herstellung eines Medikaments zur Verhütung von fetaler Infektion mit einem heterologen Virus, das zur antigenen BVDV-Typ I-Gruppe gehört.

## Revendications

1. Utilisation d'un virus recombiné BVDV (virus de la diarrhée virale bovine) de type II qui est codé par une séquence nucléotidique qui est SEQ ID NO : 1, mais dans laquelle le codon histidine H349 à l'intérieur de la région E^{RNS} est supprimé, pour la production d'un médicament destiné à la prévention de l'infection foetale par un virus hétérologue appartenant au groupe antigénique BVDV (virus de la diarrhée virale bovine) de type 1.


### SEQUENCE LISTING

<110> Boehringer Ingelheim Vetmedica GmbH
<120> Infectious bovine viral diarrhea virus clone
<130> not yet assigned
<150> DE10143813.3
   <151> 2001-09-06
<160> 28
<170> PatentIn version 3.1
<210> 1
   <211> 12332
   <212> DNA
   <213> Bovine viral diarrhea virus (BVDV)
<400> 1
   gtatacgaga ttagctaaag aactcgtata tggattggac gtcaacaaat ttttaattgg
   60
   caacgtaggg aaccttcccc tcagcgaagg ccgaaaagag gctagccatg cccttagtag
   120
   gactagcaaa agtaggggac tagcggtagc agtgagttcg ttggatggcc gaacccctga
   180
   gtacagggga gtcgtcaatg gttcgacact ccattagtcg aggagtctcg agatgccatg
   240
   tggacgaggg catgcccacg gcacatctta acccatgcgg gggttgcatg ggtgaaagcg
   300
   ctattcgtgg cgttatggac acagcctgat agggtgtagc agagacctgc tattccgcta
   360
   gtaaaaactc tgctgtacat ggcacatgga gttgttttca aatgaacttt tatacaaaac
   420
   atataaacaa aaaccagcag gcgtcgtgga acctgtttac gacgtcaacg ggcgcccact
   480
   gtttggagag agcagtgact tgcacccgca gtcaacacta aaactaccac accaacgagg
   540
   cagcgccaac atcctgacca atgctaggtc cctaccgcgg aaaggtgact gccggagagg
   600
   taatgtgtat ggaccggtga gtggcatcta tatcaaacca ggaccgatct actaccagga
   660
   ttatgtgggc cccgtctatc atagagcccc actggaacta tgtagggagg caagtatgtg
   720
   cgaaacaact aggagagttg gcagagtgac cggtagtgat gggaaattat atcatatcta
   780
   catctgcata gatgggtgta tcctcctgaa gagggcgact aggaaccaac cagaagtcct
   840
   gaaatgggta tacaacagat taaattgtcc tttatgggtc accagctgct ccgatgaagg
   900
   gagcaagggt gctacaagta agaagcagcc taagccagat aggatagaaa aaggtaagat
   960
   gaaaatagcc ccaaaagaga cagaaaaaga ttgcaaaacc agaccccccg acgcgactat
   1020
   agtagtagaa ggggttaagt accaggtgaa gaaaaaagga aaggtaaggg gaaaaaatac
   1080
   tcaagatggg ttatatcaca acaagaataa gccccctgaa tcaagaaaaa aattggaaaa
   1140
   ggcactgctg gcttgggcca tcttagcagc ggtcctgctt cagctggtaa caggagagaa
   1200
   tatcacccag tggaacttga tggacaacgg caccgaggga atacagcaag cgatgttcct
   1260
   aagaggggtg aacaggagtc tacatggaat ttggccagag aaaatttgca ccggagtacc
   1320
   aactcactta gcaacagact atgagcttaa agagatagtg gggatgatgg acgcgagtga
   1380
   gaagaccaac tacacgtgtt gcaggttgca aagacatgag tggaataaac atggttggtg
   1440
   taactggttt catatagaac cgtggatatg gttgatgaac aaaacccaaa acaacctgac
   1500
   agaagggcaa ccgcttaggg agtgtgctgt gacttgtagg tatgacaagg aaacagaatt
   1560
   gaacatcgtg acacaggcta gggacagacc tacaactctg acaggttgca agaaaggcaa
   1620
   gaatttctct ttcgcaggtg ttatactgga tgggccctgt aactttaaag tatcggttga
   1680
   agatgtgctg ttcaaggagc acgattgcgg caacatgctg caagagaccg cgatacagct
   1740
   actcgatggg gcaaccaaca ccattgaggg agcaagggta gggacggcca agttgacaac 1800
   ctggttaggg aagcaattag ggatccttgg taagaagttg gagaacaaaa gcaaagcatg 1860
   gtttggtgca catgcagcaa gtccatactg cggagtggag aggaagatcg gttacgtatg 1920
   gtatacaaaa aactgcactc cagcttgcct tccaagaaac actagaataa taggccccgg 1980
   gaaatttgat accaacgccg aagatggaaa aatactccat gagatggggg ggcacctctc 2040
   agaatttgtc ctattgtcct tggtggttct gtctgacttt gccccggaaa ccgcgagcgt 2100
   catctacttg gttctacatt ttgcgatccc gcaaagccac gttgatgtag acacatgcga 2160
   caagaaccag ctgaatttaa cggtagcaac cacagtagca gaggtcatac cagggacagt 2220
   gtggaaccta gggaagtatg tctgcataag accagactgg tggccatatg agacgacgac 2280
   agtcttcgtc atagaggaag cagggcaagt aatcaaattg atgctaaggg ccatcagaga 2340
   cttaactagg atatggaatg ctgccactac cacagctttc ttaatctttt tagtaaaagc 2400
   actgagggga caactaatcc aagggctatt gtggctgatg ctaataacag gagcacaggg 2460
   cttccctgaa tgcaaagagg gcttccaata tgccatatct aaagacagga aaatggggtt 2520
   attggggcca gagagcttaa ctacaacatg gcacctcccc accaaaaaaa tagtggattc 2580
   catggtgcat gtatggtgtg aaggaaaaga cttgaaaata ttaaaaatgt gcacaaagga 2640
   agagaggtat ctagtggctg tgcacgagag agccttatca accagtgccg agtttatgca 2700
   gatcagtgat gggacaatag gcccagacgt gatagatatg cctgatgact ttgagtttgg 2760
   actctgccct tgtgactcaa aaccagtgat aaagggcaaa tttaatgcca gcttactgaa
   2820
   tggaccagct ttccagatgg tatgcccaca ggggtggact ggtacaatag aatgcaccct
   2880
   agcgaaccaa gacaccttgg acacaactgt cattaggaca tatagaagaa ctaccccatt
   2940
   tcagcggaga aaatggtgta cctatgaaaa aataataggg gaagatatct atgaatgcat
   3000
   tctaggtgga aactggacat gcataaccgg tgaccatagc aggttgaaag acggacctat
   3060
   caagaagtgt aagtggtgtg gccatgactt cgtcaactca gaggggctac cacactaccc
   3120
   aataggcaag tgcatgctca tcaacgagag tgggtacagg tatgtagatg acacctcttg
   3180
   cgataggggt ggtgtagcca tagttccatc tggcaccgta aagtgtagaa taggtaacgt
   3240
   cacggtgcaa gttatcgcta ctaacaatga tctgggaccc atgccttgca gcccagctga
   3300
   agtgatagca agtgaaggac cagtggaaaa gactgcatgc acattcaact attcaaggac
   3360
   tctacctaat aagtattatg agccaaggga ccggtacttc caacaataca tgttaaaagg
   3420
   ggagtggcaa tattggttcg acctggattc tgtagaccac cacaaagact acttctcaga
   3480
   gttcataatc atagcagtgg tcgccttgtt gggtggtaag tacgtactgt ggctcttgat
   3540
   aacatacaca atactgtctg agcagatggc tatgggtgct ggagtgaata ctgaagagat
   3600
   agtcatgata ggcaatttgc tgacagacag tgatattgag gttgtggttt atttccttct
   3660
   tctgtactta atagttaaag aggaactggc gaggaaatgg attatactgg tataccacat
   3720
   ccttgtagcc aaccctatga aaacaattgg ggtcgtctta ctaatgctag ggggagtggt
   3780
   gaaggccagc agaatcaatg ctgatgacca aagtgctatg gacccatgct ttcttctcgt
   3840
   gacaggcgta gtggctgttt tgatgatcgc tagaagagaa cctgccacat taccactgat
   3900
   tgtagcattg ctagcaataa gaacatcagg attcctactg cccgctagca ttgatgtaac
   3960
   tgtagcagta gtattaattg tacttttgtt ggctagctac ataacagact actttagata
   4020
   taaaaagtgg cttcaactct tatttagtct gatagctggt atctttatta taaggagctt
   4080
   aaaacatatc aaccagatgg aggtaccaga aatatctatg ccaagttgga gacctctagc
   4140
   tctggtcctt ttctatataa catctacagc aataaccact aattgggaca ttgacttagc
   4200
   aggcttcctg ctgcaatggg cgccagcagt gatcatgatg gctaccatgt gggcagactt
   4260
   tttgactctg atcatagtcc tgcccagtta cgagttatct aagctttact tcctaaagaa
   4320
   cgtcaggaca gacgtggaaa agaactggct cggcaaagtg aaatacagac agatcagttc
   4380
   agtttatgac atctgtgaca gtgaggaagc agtgtaccta tttccatcaa ggcataagag
   4440
   tggaagcagg ccagatttca tattaccttt tttgaaagcc gtgttaataa gctgcatcag
   4500
   cagccaatgg caagtggttt acatttctta cctaatactg gaaattacat actatatgca
   4560
   caggaaaatc atagatgagg tgtcaggagg agcaaatttt ctatcaagac tcatagcagc
   4620
   catcatagaa ttaaattggg ccatagatga tgaggaatgt aaaggactga agaaactgta
   4680
   tctcttgtca gggagagcga agaatttgat agttaaacat aaggtaagaa atgaagccgt
   4740
   ccacagatgg tttggtgagg aggaaatata cggggcaccc aaggtgatca ctatcataaa
   4800
   agctagtacc ctaagtaaaa acaggcactg cataatctgc acgatctgtg aagggaaaga
   4860
   atggaatgga gccaactgcc caaagtgtgg aagacaagga aagcccataa catgtggaat
   4920
   gacactcgca gactttgagg agaaacatta caaaaagata tttataagag aagaatcttc
   4980
   ttgtcctgtg ccttttgatc cttcttgcca ttgtaattat tttcgccacg atgggccttt
   5040
   caggaaagag tataagggtt acgtccaata cacagccaga ggacaactct ttctgaggaa
   5100
   cctaccaatt ctagcgacga agatgaagct attaatggtg ggaaacctcg gcgcagaaat
   5160
   tggcgacctg gaacatctag gatgggtact gagagggcca gccgtgtgca aaaaaattac
   5220
   caaccatgag aagtgccacg taaacatcat ggataagcta actgcatttt ttggaatcat
   5280
   gcctagaggc acgaccccta gggcacctgt gaggttcccc acagcactac taaaagtgag
   5340
   aagggggcta gagacgggat gggcttacac gcaccaagga gggatcagct cggtagacca
   5400
   tgtcacagcc ggaaaggatt tactagtgtg tgacagtatg ggcaggacca gggttgtctg
   5460
   tcatagtaac aataagatga ctgatgagac tgagtatggc atcaagaccg actcagggtg
   5520
   tcccgaaggt gcgaggtgtt acgtgctaaa cccagaagct gttaacattt ctggcacaaa
   5580
   aggagctatg gtacacctcc agaaaacggg gggggagttc acatgtgtca ctgcctcagg
   5640
   gaccccggct ttcttcgatc tgaaaaatct aaaaggctgg tccgggctac caatttttga
   5700
   agcatccagt ggcagggtgg ttggtagggt gaaagtcggc aagaatgagg attccaagcc
   5760
   caccaaacta atgagcggaa tccagacagt gtctaagaac cagacagacc tagcggacat
   5820
   cgtaaaaaaa ttgactagta tgaacagagg agagttcaaa cagataacat tagccactgg
   5880
   ggcaggaaaa actacggaac tgccaaggtc cgtcatagag gagataggga ggcacaaaag
   5940
   ggtcttagtc ctgataccat tgagagcagc agcagagtca gtgtatcagt atatgagagt
   6000
   gaagtaccca agtatatctt tcaatttgag aataggagat atgaaggaag gtgacatggc
   6060
   cactggtatc acctacgcct catatgggta cttttgtcag cttcctcagc ccaaactgag
   6120
   agctgccatg gtagagtact catatatatt cttagatgag taccactgtg ctacacccga
   6180
   gcaattagca ataattggaa agatacacag gtttgctgaa aatcttagag tggtagcaat
   6240
   gacagcaacc ccagctggaa cggtcacaac gactggtcag aaacacccta tagaggagtt
   6300
   catagcccca gaggtgatga aaggtgaaga tctaggtagt gaatacttgg atattgcagg
   6360
   gttgaagata ccgactgaag agatgaaagg caacatgctc gtgttcgcgc caactaggaa
   6420
   catggcagta gaaacagcta agaaattgaa ggctaaggga tacaactctg gatactatta
   6480
   cagtggggaa aacccagaga acttgagggt ggtaacctcg caatccccgt atgtggtagt
   6540
   agccaccaat gccatagagt caggtgtgac attaccagac ttagacacag ttgtagacac
   6600
   tggactaaag tgtgagaaga gggtgaggat ttcttcaaaa atgcccttca ttgtaacagg
   6660
   acttaagaga atggcagtca caatcggaga gcaagcccag cgcaggggta gagtaggaag
   6720
   agtcaagcca ggtaggtact ataggagtca agaaacagct tcagggtcaa aagattacca
   6780
   ttacgaccta ctgcaagccc agaggtacgg aatagaagat ggaattaatg taacaaagtc
   6840
   attcagggag atgaactatg attggagcct ttacgaagag gacagcttga tgataactca
   6900
   actcgaggtc cttaacaacc tccttatatc agaagacctg cctgccgcag tgaagaacat
   6960
   catggcccgg accgatcacc cagaacccat acaactggcc tataacagtt atgaaaacca
   7020
   aattccagtg ctgttcccaa agatcaaaaa tggtgaggtg acagacagtt atgagaatta
   7080
   cacatatctc aatgcaagaa aattaggaga ggacgtgccg gcatatgtgt acgccacaga
   7140
   ggatgaggat ctagcagtgg atcttctggg tatggattgg ccggacccag gcaaccaaca
   7200
   ggtggtagag acagggaggg cattaaaaca agtaactggc ttatccacag cagaaaacgc
   7260
   cctcttgata gccctattcg gctacgtcgg gtaccagaca ctttcaaaaa ggcacatacc
   7320
   catgattact gacatctata cacttgaaga ccacaggctt gaggacacaa cccacctcca
   7380
   gtttgcccca aacgctataa ggaccgacgg caaggactca gagttgaagg aattagctgt
   7440
   gggagacctt gataaatatg tggacgcact ggtagactac tccaaacaag ggatgaaatt
   7500
   catcaaagtc caagctgaaa aggtcagaga ctcccagtct acgaaggaag gcttgcaaac
   7560
   cattaaggag tatgtggata agtttataca atcactaaca gagaataagg aggagatcat
   7620
   caggtatgga ctatggggag ttcacacggc actctacaaa agcttggcag cgagactggg
   7680
   gcatgaaaca gcttttgcaa ctttagtggt aaaatggttg gcttttgggg gcgaaacggt
   7740
   atctgctcac atcaagcaag tagcagttga tctagtagta tattatatca tcaacaaacc
   7800
   atcttttcct ggagatacag agacccaaca agaggggagg aagtttgtgg ctagtctttt
   7860
   tatatctgca ctagcaacat acacatataa aacctggaat tacaacaatc tgcaacgggt
   7920
   tgtcgaacct gccttagctt acctcccata tgctacaagt gccttgaagt tgttcacacc
   7980
   cacaagatta gagagtgtgg tcatactcag ttctacaatt tacaagacat acctctctat
   8040
   aaggaagggt aagagtgacg gcttgttagg tacaggcata agtgcagcca tggagatctt
   8100
   aaaccaaaac ccaatctcag taggtatatc tgtgatgctg ggggtaggtg ccatcgccgc
   8160
   ccataatgca atagaatcta gtgaacagaa aagaactttg ctgatgaagg tctttgtaaa
   8220
   aaacttctta gaccaagcag caacagatga gctagtcaaa gagaaccctg aaaaaataat
   8280
   catggctcta tttgaagcag tccagaccat aggaaacccc ctaagactca tctaccatct
   8340
   gtacggggtg tactataagg ggtgggaagc aaaagaactc gcagagaaaa ctgctggccg
   8400
   caacttattc acattgatca tgtttgaggc ctttgagctt ttaggtatgg actcagaagg
   8460
   aaagataaga aacttgtcag gcaactacat actggactta atcttcaact tgcataataa
   8520
   attaaacaag gggctcaaaa aactagtcct tgggtgggct cctgcacctt tgagctgtga
   8580
   ttggacacca agtgatgaga gaataagcct acctcataac aactacttaa gggtagaaac
   8640
   caggtgtcct tgtggctatg agatgaaggc aataaaaaat gttgctggta aattgacaaa
   8700
   agttgaagaa aaggggtcct tcctatgcag gaatagatta gggagaggac ctccaaactt
   8760
   caaagtaaca aagttctatg atgataactt gatagaagtc aagccagtag ctaggctaga
   8820
   aggccaggtg gacctctatt acaagggagt aacagctaag ttagactaca acaatgggaa
   8880
   agtactgtta gctaccaaca agtgggaggt ggaccacgct ttcctgacca gactagtaaa
   8940
   gaagcacaca gggataggtt ttaaaggtgc atatttgggt gaccgaccag accatcaaga
   9000
   tcttgtcgat agagattgtg caactataac gaagaactca gtacagttcc taaaaatgaa
   9060
   gaagggttgc gctttcacat atgacctaac aatctctaac cttgtcaggc ttattgaact
   9120
   agtccataag aataatttac aagaaagaga gatccctacc gtgacagtaa ctacttggct
   9180
   tgcatattct tttgtcaatg aagacctggg gactatcaag cctgtattgg gggagaaagt
   9240
   catcccagaa ccccccgagg agttgagtct ccaacccacc gtgagactag tcaccactga
   9300
   aacagcaata accataacag gggaggctga agtgatgacg acagggatca caccagtggt
   9360
   agagatgaaa gaagaacctc agctggacca ccagtcaact accctaaagg tagggttgaa
   9420
   ggaaggggaa tatccagggc caggagttaa ccctaaccat ttagcagagg tgatagatga
   9480
   gaaagatgac aggccttttg tcctaatcat cggtaacaaa ggttctacct cgaacagagc
   9540
   aagaacggcc aagaatatac ggctgtacaa aggaaacaac ccaagagaga tcagggatct
   9600
   gatgagccaa ggaagaatat tgacggttgc tctaaaagag ttggacccgg aattaaaaga
   9660
   attagtagat tacaagggga cctttctcaa tagggaagct ttagaagccc taagcttagg
   9720
   taagccaatc aagaggaaaa ccacaacagc aatgatcagg aggttaatag agccagaggt
   9780
   tgaggaggaa ctaccagatt ggttccaagc ggaagaaccc ctatttttgg aagcaaaaat
   9840
   acagaatgac ttataccacc taattggcag tgtagatagt ataaaaagca aagcaaagga
   9900
   attaggggcc acagataaca caaagatagt gaaggaagtt ggggctagga cctatacgat
   9960
   gaaattgagc agctggagca cacaagttac aaaaaaacag atgagtctag cccctctctt
   10020
   tgaagagctg ttattaaagt gccctccatg tagtaaaatt tcaaagggac atatggtgtc
   10080
   agcataccaa ctggctcaag gaaactggga acccctcggg tgtggggtct atatgggaac
   10140
   cataccagct aggcgtctca agatccaccc ttatgaggct taccttaaac tcaaagagct
   10200
   ggtggaagtt gaatcttcga gggccactgc aaaagaatcc atcataagag aacataacac
   10260
   ctggatcctg cggaaggtga gacatgaagg gaacctaaga accaaatcaa tgatcaaccc
   10320
   tgggaaaata tcagatcagc tatgcagaga tggacacaaa agaaacatat ataataagat
   10380
   cataggctca acaatggcct ctgctggtat taggctggag aaactgccag tagtccgagc
   10440
   ccaaactgac acaaccagtt tccaccaagc cataagagaa aaaattgata aaacagaaaa
   10500
   caagcagacc cctgaattgc atgaagaact aatgaaggtc ttcgactgct taaagatccc
   10560
   agagctgaag gaatcgtatg atgaagtttc atgggaacaa ttagaagccg ggataaaccg
   10620
   taagggtgca gcaggctatc tagagagcaa gaacataggg gaagtcctag acacagagaa
   10680
   acacatagta gagcagctga tcaaggatct gaggaagggg aagaagatta ggtactatga
   10740
   aacagccatc cccaagaatg agaagagaga cgtcagcgac gactgggaag ccggagagtt
   10800
   cgttgatgaa aagaaaccaa gagtaatcca gtacccggac gccaaggtga gactggccat
   10860
   tacaaaagtg atgtacaaat gggtaaagca aaaaccagtg gtgatacccg gctatgaagg
   10920
   taaaacacct ctatttgaca tattcaacaa agtgaagaag gaatgggatt cattccagga
   10980
   ccccgtagca gtgagctttg acaccaaagc gtgggataca caagtcacca gtagagacct
   11040
   aatgttgata aaggatatcc agaaatatta tttcaagaga agtatacaca aatttttaga
   11100
   tacaataaca gaacacatgg tggaggtacc tgtcattaca gcagacggtg aagtttacat
   11160
   aaggaatggt cagaggggta gtggccaacc cgacacaagt gctggtaata gtatgttgaa
   11220
   tgtcctaacc atgatatatg ctttctgtaa aagtacaggc ataccttaca ggggattcag
   11280
   cagagtggca agaatccatg tgtgtggtga tgatggcttt ttgataacag agagaggact
   11340
   gggactgaaa ttctctgaga agggtatgca gatattacat gaggccggga agccccagaa
   11400
   aataactgaa ggggacaaaa tgaaagtggc atacagattc gaggacatag agttttgttc
   11460
   ccatactccc gtgccagtca gatgggcaga taacaccagt agttacatgg cagggaggag
   11520
   cacagccact atactagcta agatggcaac caggctggat tccagcggag agaggggtag
   11580
   cacagcttat gagaaggccg tagccttcag cttccttttg atgtactcat ggaatcccgt
   11640
   agttagaagg atctgcttac tggtgttgtc acagtttcca gaaatatccc catccaaaaa
   11700
   cacaatatac tactaccaag gggatcccat agctgcgtac agagaagtga tagggaaaca
   11760
   gctgtgtgaa ctgaaaagaa caggatttga gaagctggct ggtctgaatt tgagtatgac
   11820
   cactctaggc atctggacaa aacatactag taaaagacta atccaagcct gtgtagaaat
   11880
   aggtaagaga gaaggtacct ggttagttaa tgctgacaga ctgattgcag gaaagactgg
   11940
   gaagttttac atcccaagca ctggtgtcac tctgttggga aaacactatg aggaaattaa
   12000
   cttaaagcaa aaggcggcac aaccgccgat agagggggtt gacagatata agttgggccc
   12060
   catagttaat gttatcttga gaaggctgag ggtgatgctg atgacagttg ccagcggaag
   12120
   ctggtgaatc cgtccggagc gtcgtgccct cactcaaggt ttttaattgt aaatattgta
   12180
   aatagacagc taagatattt attgtagttg gatagtaatg cagtgatagt aaatacccca
   12240
   atttaacact acctccaatg cactaagcac tttagctgtg tgaggttaac tcgacgtcca
   12300
   cggttggact agggaagacc tctaacagcc cc
   12332
<210> 2
   <211> 24
   <212> DNA
   <213> PCR Primer
<400> 2
   ctccatgtgc catgtacagc agag
   24
<210> 3
   <211> 24
   <212> DNA
   <213> PCR Primer
<400> 3
   ctcgtccaca tggcatctcg agac
   24
<210> 4
   <211> 20
   <212> DNA
   <213> PCR Primer
<400> 4
   gcactggtgt cactctgttg
   20
<210> 5
   <211> 25
   <212> DNA
   <213> PCR Primer
<400> 5
   gagaaggctg agggtgatgc tgatg
   25
<210> 6
   <211> 21
   <212> DNA
   <213> PCR Primer
<400> 6
   gactttccgc ttctttttag g
   21
<210> 7
   <211> 20
   <212> DNA
   <213> PCR Primer
<400> 7
   ggagagaata tcacccagtg
   20
<210> 8
   <211> 24
   <212> DNA
   <213> PCR Primer
<400> 8
   ctccactccg cagtatggac ttgc
   24
<210> 9
   <211> 21
   <212> DNA
   <213> oligonucleotide
<400> 9
   cctaaaaaga agcggaaagt c
   21
<210> 10
   <211> 21
   <212> DNA
   <213> oligonucleotide
<400> 10
   gttgacatgg catttttcgt g
   21
<210> 11
   <211> 22
   <212> DNA
   <213> oligonucleotide
<400> 11
   cctcttatac gttctcacaa cg
   22
<210> 12
   <211> 22
   <212> DNA
   <213> oligonucleotide
<400> 12
   gcatccatca taccrtgatg at
   22
<210> 13
   <211> 22
   <212> DNA
   <213> oligonucleotide
<400> 13
   gcatccatca tcccrtgatg at
   22
<210> 14
   <211> 22
   <212> DNA
   <213> oligonucleotide
<400> 14
   gcatccatca tgccrtgatg at
   22
<210> 15
   <211> 22
   <212> DNA
   <213> oligonucleotide
<400> 15
   gcatccatca ttccrtgatg at
   22
<210> 16
   <211> 24
   <212> DNA
   <213> oligonucleotide
<400> 16
   caaatctctg atcagttgtt ccac
   24
<210> 17
   <211> 17
   <212> DNA
   <213> oligonucleotide
<400> 17
   ttgcacacgg caggtcc
   17
<210> 18
   <211> 35
   <212> DNA
   <213> oligonucleotide
<400> 18
   gtcccccggg ggctgttaag ggttttccta gtcca
   35
<210> 19
   <211> 25
   <212> DNA
   <213> PCR Primer
<400> 19
   gatgtagaca catgcgacaa gaacc
   25
<210> 20
   <211> 20
   <212> DNA
   <213> PCR Primer
<400> 20
   gcttccactc ttatgccttg
   20
<210> 21
   <211> 78
   <212> DNA
   <213> PCR Primer
<400> 21
   gctctagacg gccgtaatac gactcactat aggtatacga gattagctaa agaactcgta
   60
   tatggattgg acgtcaac
   78
<210> 22
   <211> 32
   <212> DNA
   <213> PCR Primer
<400> 22
   gacggccgta atacgactca ctatagtata cg
   32
<210> 23
   <211> 24
   <212> DNA
   <213> PCR Primer
<400> 23
   cctaaccatg atatatgcct tctg
   24
<210> 24
   <211> 36
   <212> DNA
   <213> PCR Primer
<400> 24
   cggaattcgc ccgggctgtt agaggtcttc cctagt
   36
<210> 25
   <211> 24
   <212> DNA
   <213> oligonucleotide
<400> 25
   gagtggaata aaggttggtg taac
   24
<210> 26
   <211> 24
   <212> DNA
   <213> oligonucleotide
<400> 26
   gttacaccaa cctttattcc actc
   24
<210> 27
   <211> 27
   <212> DNA
   <213> oligonucleotide
<400> 27
   aacaggagtc tattaggaat ttggcca 27
<210> 28
   <211> 27
   <212> DNA
   <213> oligonucleotide
<400> 28
   tggccaaatt cctaatagac tcctgtt 27